(19) Europäisches Patentamt
European Patent Office
Office européen des brevets

(11) Numéro de publication : **0 305 289 B1**

# (12) FASCICULE DE BREVET EUROPEEN

(45) Date de publication du fascicule du brevet : **16.10.91 Bulletin 91/42**

(51) Int. Cl.$^5$ : **C07C 69/757**, C07D 317/30

(21) Numéro de dépôt : **88402147.8**

(22) Date de dépôt : **24.08.88**

(54) **Procédé énantiosélectif pour préparer des dérivés de l'aldéhyde hémicaronique de structure trans ou cis et intermédiaires obtenus.**

(30) Priorité : **24.08.87 FR 8711849**

(43) Date de publication de la demande : **01.03.89 Bulletin 89/09**

(45) Mention de la délivrance du brevet : **16.10.91 Bulletin 91/42**

(84) Etats contractants désignés : **AT BE CH DE ES FR GB IT LI LU NL SE**

(56) Documents cités : **DE-A- 3 229 537**

(73) Titulaire : **ROUSSEL-UCLAF**
**35, Boulevard des Invalides**
**F-75007 Paris (FR)**

(72) Inventeur : **Krief, Alain**
**34, Rue Tienne aux Cloches**
**B-5150 Wepion (BE)**
Inventeur : **Dumont, Willy**
**18, Rue des Bruyères**
**B-5190 Salet - Anhee (BE)**

(74) Mandataire : **Tonnellier, Marie-José et al**
**111, route de Noisy B.P. no 9**
**F-93230 Romainville (FR)**

**EP 0 305 289 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

**Description**

La présente invention a pour objet un nouveau procédé énantiosélectif pour préparer des dérivés de l'aldéhyde hémicaronique de structure trans ou cis et de nouveaux intermédiaires.

L'invention a ainsi pour objet un procédé de préparation des composés de formule (I) :

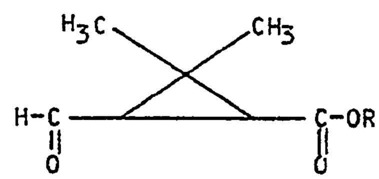

de structure cis ou trans, racémiques ou optiquement actifs, dans laquelle R représente un atome d'hydrogène, un radical alcoyle renfermant de 1 à 4 atomes de carbone ou un radical aryle renfermant jusqu'à 12 atomes de carbone, caractérisé en ce que l'on soumet à l'action d'un agent de réduction ménagée un diester de formule (II) :

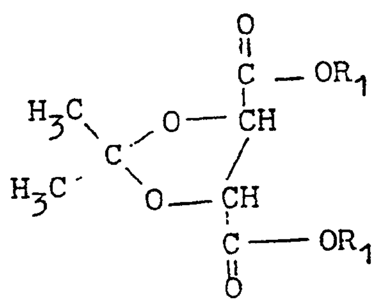

sous l'une quelconque de ses formes isomères dédoublées ou racémiques, dans laquelle $R_1$ représente un radical alcoyle renfermant de 1 à 4 atomes de carbone, un radical aryle renfermant jusqu'à 12 atomes de carbone ou un radical aralcoyle renfermant jusqu'à 8 atomes de carbone, pour obtenir un précurseur d'un dialdéhyde de formule (III) :

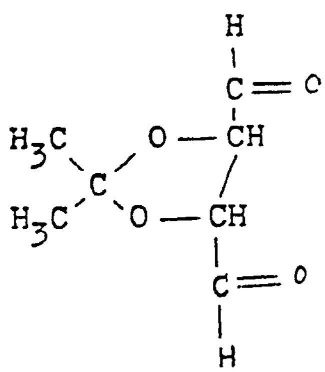

(III)

que l'on soumet à l'action d'un réactif de Wittig-Emmons-Horner de formule :

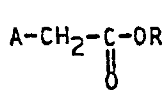

dans laquelle R est défini comme précédemment et A représente le reste organique phosphoré dudit réactif, pour obtenir le composé de formule (IV) :

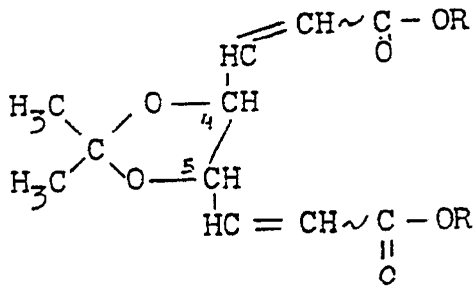

(IV)

dans laquelle R est défini comme précédemment et les traits ondulés schématisent une configuration cis ou trans, que l'on soumet à l'action d'un agent de gem-diméthyl cyclopropanation pour obtenir soit le composé de formule (V) :

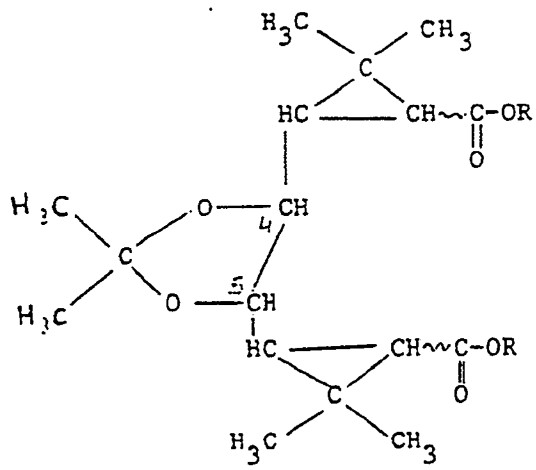

(V)

dans laquelle R et les traits ondulés sont définis comme précédemment, soit le composé de formule (Va) :

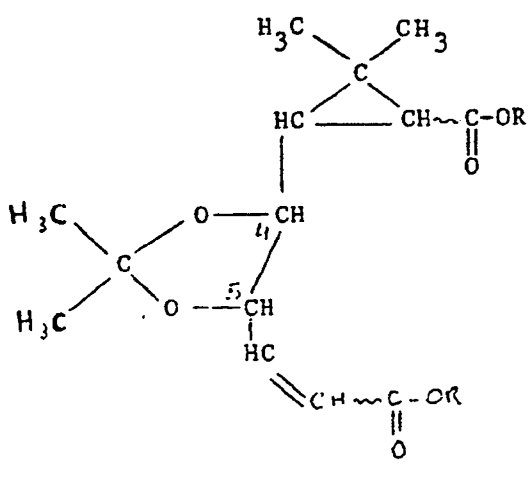

(Va)

dans laquelle R et les traits ondulés sont définis comme précédemment, composé de formule Va que, le cas échéant, l'on soumet à un agent de gem-diméthyl cyclopropanation, pour obtenir le composé de formule (V) définie précédemment, produit de formule (V) ou (Va) dont soit l'on hydrolyse le reste dioxolanne, pour obtenir respectivement ou bien le composé de formule (VI) :

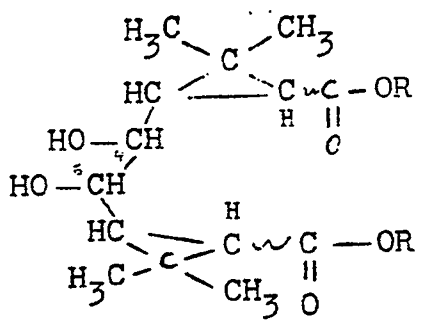

(VI)

dans laquelle R et les traits ondulés sont définis, comme précédemment, ou bien le composé de formule (VIa):

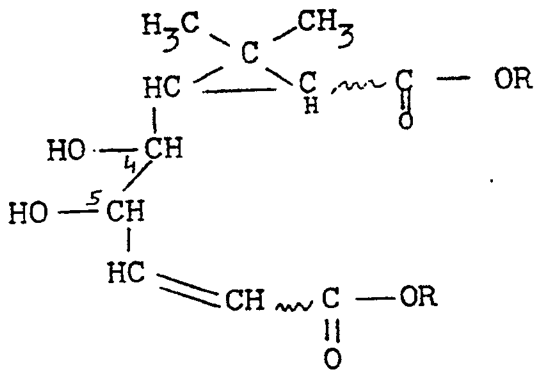

(VIa)

dans laquelle R et les traits ondulés sont définis comme précédemment, composé de formule (VI) ou (VIa), dont on clive la liaison 4, 5 pour obtenir le composé de formule (I) attendu, soit l'on hydrolyse le reste dioxolanne et clive simultanément la liaison 4, 5, pour obtenir le composé de formule (I) attendu.

R peut représenter un radical méthyle, éthyle, propyle linéaire ou ramifié ou butyle linéaire ou ramifié.

Lorsque $R_1$ représente un radical alcoyle, il peut prendre les valeurs de R définies ci-dessus.

Lorsque $R_1$ représente un radical aryle, il peut s'agir par exemple d'un radical phényle, tolyle ou naphtyle.

Lorsque $R_1$ représente un radical aralcoyle, il peut s'agir par exemple d'un radical benzyle ou phénéthyle.

A représente le reste organique phosphoré d'un réactif de Wittig-Emmons-Horner, à savoir le reste d'un phosphonate, d'un sel de triarylphosphonium ou d'un oxyde de diarylphosphine.

Dans des conditions préférentielles d'exécution du procédé selon l'invention :

— l'agent de réduction ménagée est l'hydrure de diisobutylaluminium ;

— le réactif de Wittig, Emmons ou Horner est un réactif de formule

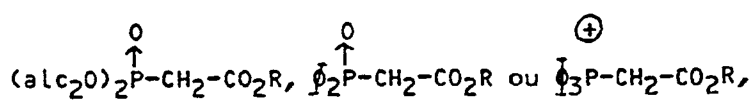

dans laquelle R est défini comme précédemment, $alc_2$ représente un radical méthyle, éthyle, propyle linéaire ou ramifié ou butyle linéaire ou ramifié et Φ représente un radical phényle ;

— l'agent de gem-diméthyl cyclopropanation est un réactif de formule :

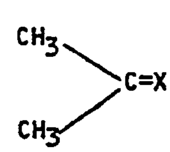

dans laquelle X représente un reste P $(Ar)_3$, S $(Ar)_2$ ou

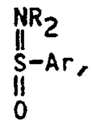

dans laquelle Ar représente un radical aryle et notamment phényle et $R_2$ représente un atome d'hydrogène ou un radical alcoyle renfermant de 1 à 4 atomes de carbone ;

— l'agent d'hydrolyse du reste dioxolanne est un agent acide minéral ou organique, tel que l'acide chlorhydrique, sulfurique, acétique, perchlorique, paratoluène sulfonique ;

— l'agent de clivage de la liaison 4, 5 est un agent oxydant tel qu'un périodate, le tétracétate de plomb ou le permanganate de potassium ;

— l'agent susceptible d'hydrolyser le reste dioxolanne et de cliver la liaison 4, 5 est un agent oxydant tel que l'acide périodique ou un périodate en présence d'acide sulfurique.

Le réactif de formule

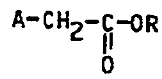

peut engendrer soit un diester de géométrie E-E, soit un mélange séparable par les moyens classiques de diesters de géométries (Z-Z) et (Z-E).

Le réactif de gem-diméthyl cyclopropanation réagit sur le composé de formule (IV) pour conduire selon la quantité de réactif utilisée, soit à un composé dicyclopropanique de formule (V), soit à un composé monocyclopropanique de formule (Va), lequel est à son tour transformable en composé de formule (V).

L'invention a notamment pour objet un procédé de préparation des composés de formule ($I_A$) :

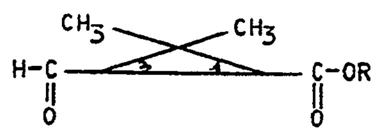

($I_A$)

de structure trans, racémiques ou optiquement actifs, dans laquelle R est défini comme précédemment, caractérisé en ce que l'on soumet à l'action d'un agent de réduction ménagée, un diester de formule (II), telle que définie précédemment, sous l'une quelconque de ses formes isomères dédoublées ou racémiques, pour obtenir un précurseur d'un dialdéhyde de formule (III) telle que définie précédemment, de structure isomérique correspondante, que l'on soumet à l'action d'un réactif de formule :

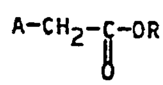

dans laquelle A et R sont définis comme précédemment, pour obtenir le composé de formule (IV') :

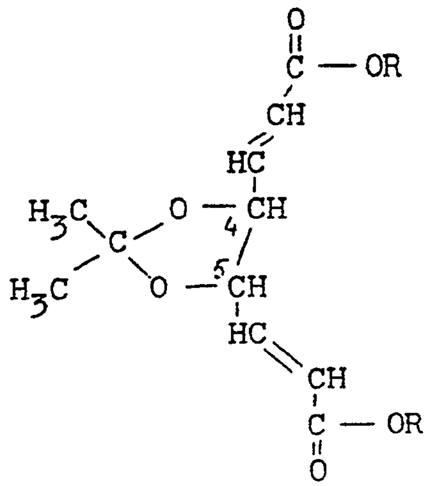

(IV')

dans laquelle R est défini comme précédemment et la géométrie des doubles liaisons est trans, que l'on soumet à l'action d'un agent de gem-diméthyl cyclopropanation pour obtenir le composé de formule (V') :

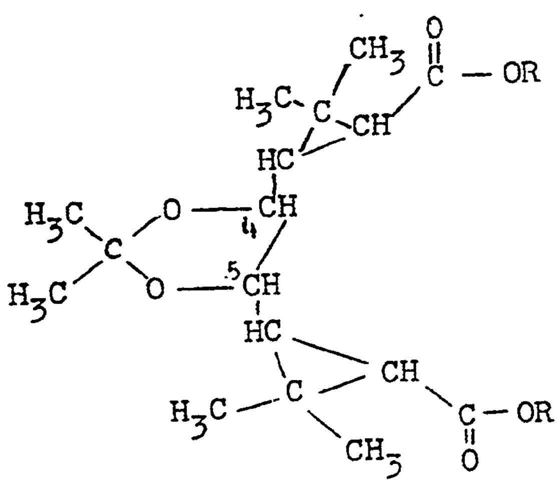

(V')

dans laquelle R est défini comme précédemment et la configuration des cycle cyclopropaniques est trans, dont soit l'on hydrolyse le reste dioxolanne pour obtenir le composé de formule (VI') :

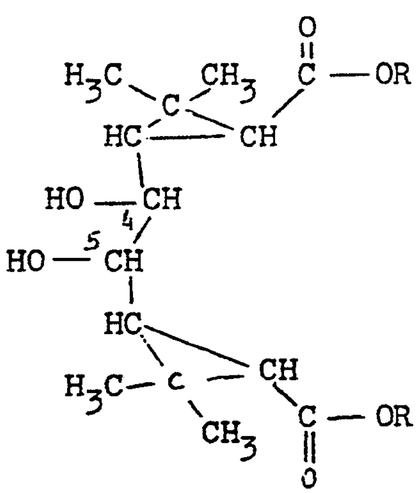

(VI')

dans laquelle R et la configuration des cycles cyclopropaniques sont définies comme précédemment, et clive la liaison 4, 5, pour obtenir le composé de formule ($I_A$) correspondant, soit l'on hydrolyse le reste dioxolanne

et clive simultanément la liaison 4, 5, pour obtenir le composé de formule ($I_A$) attendu.

L'invention a plus particulièrement pour objet un procédé de préparation des composés de formule ($I_A$) de configuration 1R trans, caractérisé en ce que l'on soumet à l'action de l'hydrure de diisobutylaluminium un diester de formule ($II_1$) :

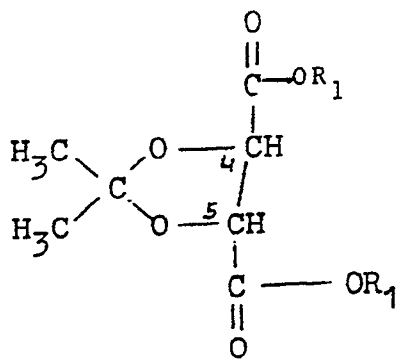

($II_1$)

de configuration (4R, 5R), dans laquelle $R_1$ est défini comme précédemment, pour obtenir le précurseur correspondant du dialdéhyde de formule ($III_1$) :

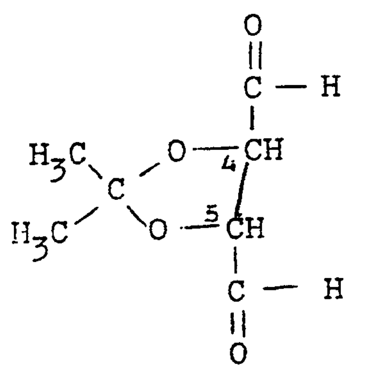

($III_1$)

de configuration (4R, 5R), que l'on soumet à l'action d'un réactif de formule :

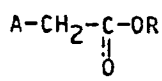

dans laquelle A et R sont définis comme précédemment, pour obtenir le composé de formule ($IV'_1$) :

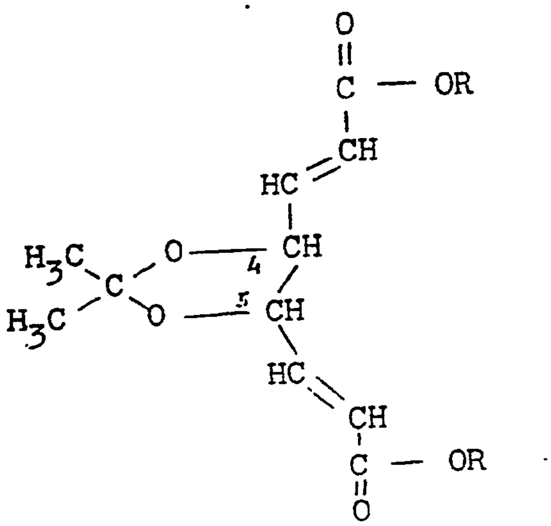

(IV'₁)

de configuration (4S, 5S), dans laquelle R est défini comme précédemment et la géométrie des doubles liaisons est trans, que l'on soumet à l'action d'un isopropylidène triphénylphosphorane pour obtenir le composé de formule ($V'_1$) :

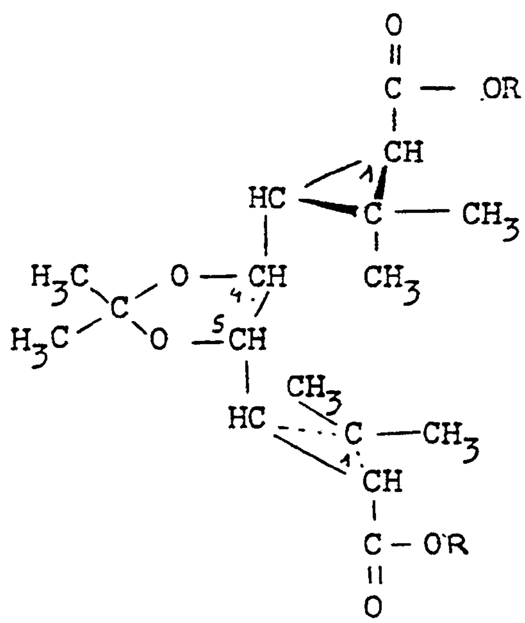

($V'_1$)

de configuration (4S, 5S) et (1R trans) au niveau des cycles cylopropanes, dans laquelle R est défini comme précédemment, dont soit l'on hydrolyse le reste dioxolanne, pour obtenir le composé de formule ($VI'_1$) :

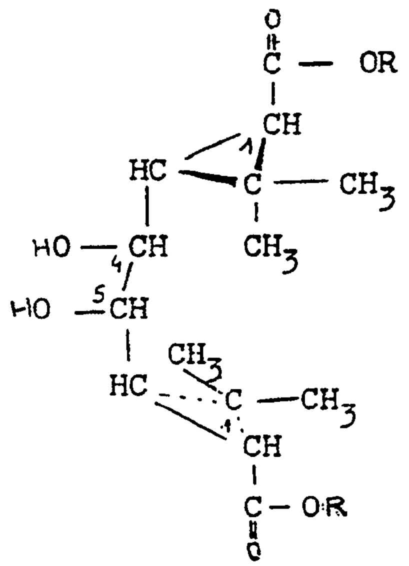

(VI'₁)

de configuration (4S, 5S) et (1R trans) au niveau des cycles cyclopropanes, dans laquelle R est défini comme précédemment et clive la liaison 4, 5 par action d'un agent oxydant, pour obtenir le composé de formule ($I_A$) de configuration (1R trans), soit l'on hydrolyse le reste dioxolanne et clive simultanément la liaison 4, 5 pour obtenir le composé de formule ($I_A$) de configuration (1R trans).

L'invention a aussi notamment pour objet un procédé de préparation des composés de formule ($I_B$) :

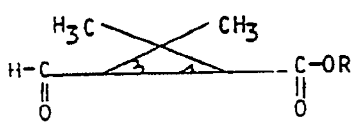

($I_B$)

de structure cis, racémiques ou optiquement actifs, dans laquelle R est défini comme précédemment, caractérisé en ce que l'on soumet à l'action d'un agent de réduction ménagée, un diester de formule (II), telle que définie précédemment, sous l'une quelconque de ses formes isomères dédoublées ou racémiques, pour obtenir un précurseur d'un dialdéhyde de formule (III) telle que définie précédemment, de structure isomérique corrrespondante, que l'on soumet à l'action d'un réactif de formule :

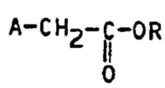

dans laquelle A et R sont définis comme précédemment, pour obtenir le composé de formule (IV") :

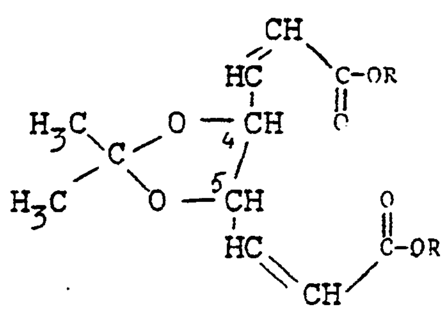

(IV")

dans laquelle R est défini comme précédemment et la géométrie des doubles liaisons est cis, que l'on soumet à l'action d'un isopropylidène diphényl sulfurane pour obtenir le composé de formule (V") :

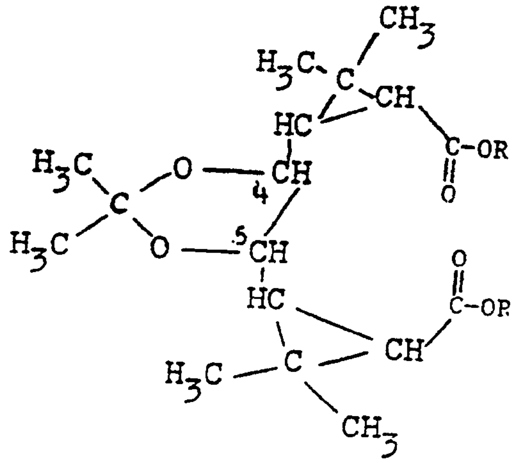

(V")

dans laquelle R est défini comme précédemment et la configuration des cycles cyclopropaniques est cis, dont soit l'on hydrolyse le reste dioxolanne pour obtenir le composé de formule (VI") :

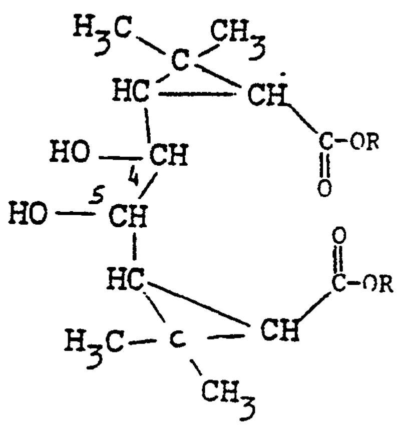

(VI")

dans laquelle R et la configuration des cycles cyclopropaniques sont définies comme précédemment, et clive la liaison 4, 5, pour obtenir le composé de formule ($I_B$) correspondant, soit l'on hydrolyse le reste dioxolanne et clive simultanément la liaison 4, 5, pour obtenir le composé de formule ($I_B$) attendu.

L'invention a plus particulièrement pour objet un procédé de préparation des composés de formule ($I_B$) de configuration 1R cis, caractérisé en ce que l'on soumet à l'action de l'hydrure de diisobutylaluminium un diester de formule ($II_1$) :

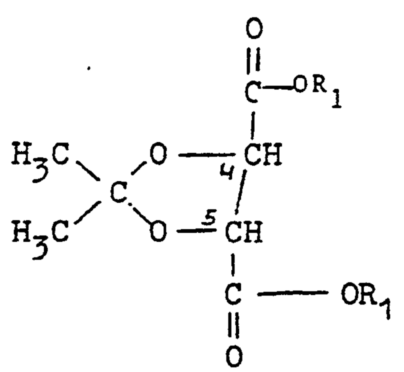

($II_1$)

de configuration (4R, 5R), dans laquelle $R_1$ est défini comme précédemment, pour obtenir le précurseur correspondant du dialdéhyde de formule ($III_1$) :

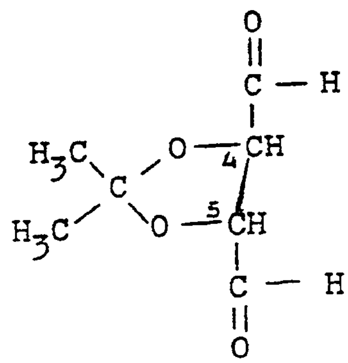

$(III_1)$

de configuration (4R, 5R), que l'on soumet à l'action d'un réactif de formule :

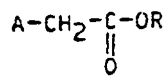

dans laquelle A et R sont définis comme précédemment, pour obtenir le composé de formule $(IV''_1)$ :

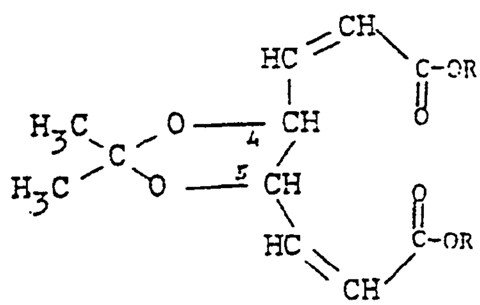

$(IV''_1)$

de configuration (4S, 5S), dans laquelle R est défini comme précédemment et la géométrie des doubles liaisons est cis, que l'on soumet à l'action d'un isopropylidène diphénylsulfurane pour obtenir le composé de formule $(V''_1)$ :

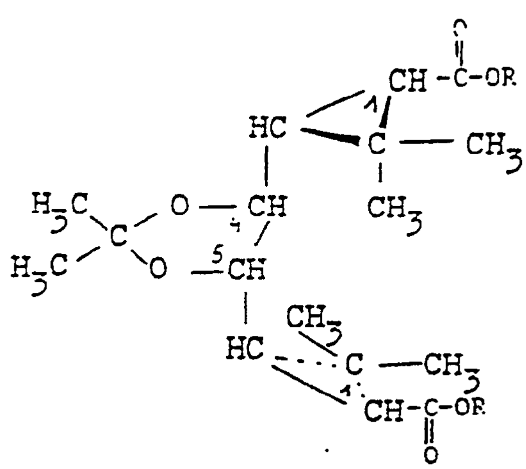

$(V''_1)$

de configuration (4S, 5S) et (1R cis) au niveau des cycles cylopropanes, dans laquelle R est défini comme précédemment, dont soit l'on hydrolyse le reste dioxolanne, pour obtenir le composé de formule $(VI''_1)$ :

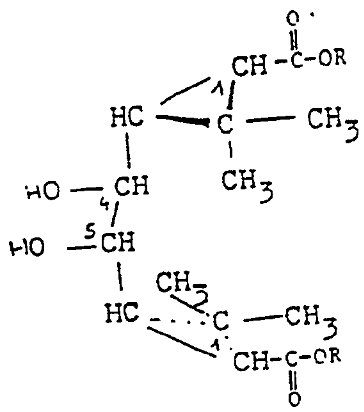

(VI"$_1$)

de configuration (4S, 5S) et (1R cis) au niveau des cycles cyclopropanes, dans laquelle R est défini comme précédemment et clive la liaison 4, 5 par action d'un agent oxydant, pour obtenir le composé de formule ($I_B$) de configuration (1R cis), soit l'on hydrolyse le reste dioxolanne et clive simultanément la liaison 4, 5 pour obtenir le composé de formule ($I_B$) de configuration (1R cis).

Outre les composés de formule I de structure 1R trans et 1R cis selon la configuration en position 4, 5 du composé de départ de formule (II) et selon la configuration de la double liaison des composés de formule (IV), issues de l'action du réactif

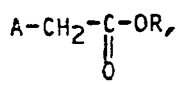

le procédé de l'invention permet permet l'accès énantiosélectif à toutes le structures isomériques possibles de formule (I).

Dans les tableaux ci-après, ont été représentées les configurations des composés de formule (I) obtenus de géométrie cis ou trans et de géométrie cis ainsi que des intermédiaires isolés, au départ de composés chiraux de formule (II) :

| Isomère | X de réactif cyclopropanation | Composé (II) 4 5 | Composé (IV) 2 4 5 6 | Composé (Va) 2 3 4 5 6 | Composé (V) 2 3 4 5 6 7 | Composé (I) |
|---|---|---|---|---|---|---|
| 1 | $P\Phi_3$ | R R | E S S E | R R S S E | R R S S R R | 1R trans |
| 2 | $P\Phi_3$ | R R | Z S S Z | S S S S E | S S S S R R | dl trans |
| 3 | $S\Phi_2$ | R R | Z S S Z | S S S S Z | S S S S S S | 1S trans |
| 4 | $P\Phi_3$ ou $S\Phi_2$ | R R | E S S Z | S S S S E | S S S S R R | dl trans |
| 5 | $P\Phi_3$ | S S | E R R E | S S R R E | S S R R S S | 1S trans |
| 6 | $P\Phi_3$ | S S | Z R R Z | R R R R E | R R R R S S | dl trans |
| 7 | $S\Phi_2$ | S S | Z R R Z | R R R R Z | R R R R R R | 1R trans |
| 8 | $P\Phi_3$ ou $S\Phi_2$ | S S | E R R Z | R R R R E | R R R R S S | dl trans |
| 1 | $S\Phi_2$ | R R | Z S S Z | R S S S Z | R S S S S R | 1R cis |
| 5 | $S\Phi_2$ | S S | Z R R Z | S R R R Z | S R R R R S | 1S cis |

L'invention a également pour objet les composés de formule IV, V, Va, VI et VIa obtenus lors de la mise en oeuvre du procédé de préparation des composés de formule (I).

Les composés de formule (I) connus sous l'appellation d'aldéhyde hémicaronique sont des intermédiaires utiles dans la synthèse de l'acide chrysanthémique ou de ses analogues notamment halogénés.

L'invention a enfin pour objet, à titre de composé nouveau, le composé tel qu'obtenu par la réduction du diester de formule ($II_1$), telle que définie précédemment, par l'hydrure de diisobutylaluminium.

Les esters de formule (II) sont des composés connus, pouvant être obtenus, à partir d'un acide tartrique comme décrit ci-après dans la partie expérimentale.

Les exemples suivants illustrent l'invention, sans toutefois la limiter.

**Exemple 1 : (1R, 3R) 3-formyl 2,2-diméthyl cyclopropane carboxylate de méthyle.**

**Stade A :**

(4S, 5S) 2,2-diméthyl 1,3-dioxolanne 4,5-diyl 3,3'-dipropénate de méthyle (E).

On refroidit à −78°C sous atmosphère inerte 4,36 g de produit obtenu selon la préparation ci-après en solution dans 50 $cm^3$ de toluène, ajoute lentement 26,6 $cm^3$ d'une solution 1,5 M d'hydrure de diisobutylaluminium dans le toluène et maintient sous agitation à −78°C pendant deux heures. On ajoute goutte à goutte à la solution obtenue, une solution diéthylphosphonate de sodium dans le diméthoxyéthane dont la préparation est donnée ci-dessous et laisse le mélange réactionnel revenir à température ambiante. On agite 4 heures à 20°C, ajoute

20 $cm^3$ d'eau, extrait à l'éther, sèche et élimine les solvants sous pression réduite. On recueille 4,7 g de produit brut que l'on chromatographie sur silice (éluant hexane-acétate d'éthyle 9/1). On obtient 2,3 g de produit attendu que l'on utilise tel quel pour le stade suivant.

$[alpha]_D$ = –70,2° (c = 6 mg/ml $CHCl_3$).

La solution de diéthylphosphonate de sodium utilisée plus haut a été préparée comme suit :

— On met en suspension, sous atmosphère inerte, à 0°C, 1,4 g d'hydrure de sodium dans 20 $cm^3$ de diméthoxyéthane, ajoute 9,45 g de diéthylphosphonoacétate de méthyle en solution dans 50 $cm^3$ de diméthoxyéthane et agite 30 minutes à 20°C.

Préparation : (4R, 5R) 2,2-diméthyl 1,3-dioxolanne 4,5-dicarboxylate de méthyle.

On dissout 50,5 g d'acide tartrique (2R, 3R) dans 80 g de 2,2-diméthoxy propane, ajoute une solution comprenant 0,2 g d'acide paratoluène sulfonique dans 20 $cm^3$ de méthanol et chauffe au reflux pendant 15 heures. On ajoute 40 g de 2,2-diméthoxypropane dilué dans 225 $cm^3$ de cyclohexane et distille lentement le milieu réactionnel pendant 24 heures à 79°C. On ajoute 0,5 g de carbonate de potassium, évapore le reste du cyclohexane, et purifie par distillation fractionnée sous pression réduite.

On obtient 67 g de produit attendu Eb = 86°-91°C (0,4 mm Hg).

Spectre IR ($cm^{-1}$) :

3480-2980-2950-1750-1440-1350-1210-1160-1100-1010-860-840-810-780-750-700

Spectre de RMN ($CCl_4$) :

| | |
|---|---|
| H des méthyles : | 1,46 ppm (s) |
| H des $CO_2CH_3$ : | 3,79 ppm (s) |
| H de —CH-O : | 4,66 ppm(s) |

Stade B :

[(4S, 5S) 2,2-diméthyl 1,3-dioxolanne 4,5-diyl/ 3,3'-di-/2,2-diméthyl 1R, 3R cyclopropane carboxylate de méthyle/.

On prépare une solution (12,5 × $10^{-3}$ m) de triphénylisopropylidène phosphorane dans le tétrahydrofuranne par addition d'une solution hexanique de n-butyllithium à une suspension d'iodure d'isopropyltriphénylphosphonium dans le tétrahydrofuranne. On introduit à 0°C 20 $cm^3$ de cette solution dans 1,35 g du produit préparé au stade A, dissout dans 25 $cm^3$ de tétrahydrofuranne et maintient sous agitation 1 heure à 0°C puis 1 heure, après retour à température ambiante. On dilue le milieu réactionnel avec 10 $cm^3$ d'eau, extrait à l'éther, sèche et élimine les solvants sous pression réduite.

Après purification par chromatographie, on obtient le produit attendu avec un rendement de 50 à 60% que l'on recristallise dans le cyclohexane :

$[alpha]^{20}_D$ = –55,5° (c : 20 ; 25 $CHCl_3$)

Stade C :

[(1S, 2S) 1,2-dihydroxy-éthylène] 3,3'-di-/2,2-diméthyl 1R, 3R-cyclopropane carboxylate de méthyle/.

On dissout 180 mg du produit obtenu au stade B dans 4 $cm^3$ de tétrahydrofuranne, ajoute 5 $cm^3$ d'une solution aqueuse 2 N d'acide perchlorique et agite 8 heures à 20°C. On neutralise le milieu réactionnel avec une solution aqueuse saturée en bicarbonate de sodium, extrait à l'éther, sèche la phase organique et élimine les solvants sous pression réduite. On récupère 160 mg de produit brut que l'on utilise tel quel pour le stade suivant.

$[alpha]^{20}_D$ = –67,5° (13,96 $CHCl_3$)

Stade D :

(1R, 3R) 3-formyl 2,2-diméthyl cyclopropane carboxylate de méthyle.

On dissout 105 mg du diol obtenu au stade C dans 4 $cm^3$ de méthanol, ajuste le pH à 7 avec 2 $cm^3$ de tampon phosphate, puis ajoute en une seule fois 107 mg de périodate de sodium. On agite 30 minutes à température ambiante, filtre le résidu, le lave à l'éther, puis élimine les solvants sous pression réduite. On reprend le résidu dans l'éther, lave à l'eau, sèche, évapore les solvants sous pression réduite et recueille 82 mg de produit brut que l'on purifie par chromatographie sur silice (éluant pentane-éther éthylique 7-3). On obtient 72 mg de produit attendu.

$[alpha]_D^{20}$ = + 18,85° (18,25 acétone).

Exemple 2 : (1S, 3S) 3-formyl 2,2-diméthyl cyclopropane carboxylate de méthyle.

Stade A :

[(4S, 5S) 2,2-diméthyl 1,3-dioxolanne 4,5-diyl] 3,3'-di [2,2-diméthyl 1S, 3S cyclopropane carboxylate de méthyle].

A une solution refroidie à −78°C et placée sous gaz inerte de 0,915 g de tétrafluoroborate d'isopropyldiphénylsufonium et de 0,255 g de dichlorométhane anhydre dans 12 $cm^3$ de diméthoxyéthane anhydre, on ajoute une solution de diisopropylamidure de lithium (obtenue par traitement durant 15 minutes à −78°C de 0,325 g de diisopropylamine dans 5 $cm^3$ de diméthoxyéthane par 2,1 $cm^3$ de n-butyllithium (1,55 M dans l'hexane). On laisse agiter durant 15 minutes à −78°C puis on additionne 0,27 g de diester obtenu comme décrit au stade A de l'exemple 1, en solution dans 2 $cm^3$ de diméthoxyéthane. Le mélange réactionnel est agité durant 15 minutes à −78°C puis 45 minutes entre −65°C et −50°C. On laisse finalement réchauffer durant 15 minutes puis on ajoute 5 $cm^3$ d'eau. La phase organique est diluée dans l'éther, séparée de la phase aqueuse, lavée par l'eau et séchée. L'évaporation des solvants sous vide fournit 0,75 g de mélange réactionnel brut qui est purifié par chromatographie sur silice (éluant : pentane-éther 75-25). On obtient 0,29 g de produit attendu. F = 136-137°C.

$[alpha]_D^{20}$ = −24,7° (c = 16,6 mg/ml chloroforme).

Spectre de RMN ($CDCl_3$) :

| | |
|---|---|
| = 1-1,6 | (m avec 1 à 1,2 ; 1,24 et 1,35, (22H, $CH_3$ et H cyclopropaniques, |
| 3,2-3,44 | (m, 2H, H du dioxolane), |
| 3,6 | (s, 6H, $CO_2CH_3$). |

Stade B :

[(1S, 2S) 1,2-dihydroxy éthylène] 3,3'-di [2,2-diméthyl 1S, 3S-cyclopropane carboxylate de méthyle].

On opère de manière analogue à celle décrite au stade C de l'exemple 1 au départ de 0,29 g de produit obtenu au stade A et obtient le produit attendu utilisé à l'état brut pour le stade suivant.

Stade C :

(1S, 3S) 3-formyl 2,2-diméthyl cyclopropane carboxylate de méthyle.

On opère de manière analogue à celle décrite au stade D de l'exemple 1, au départ de 0,31 g de produit obtenu comme décrit au stade B, en utilisant 0,32 g de périodate de sodium. Après filtration, lavage à l'éther, élimination de solvant et chromatographie sur silice en éluant au mélange éther-pentane (2-8), on obtient 0,21 g de produit attendu.

$[alpha]_D^{20}$ = −18° (c = 25,5 mg/ml acétone).

**Exemple 3 : (1R, 3S) 3-formyl 2,2-diméthyl cyclopropane carboxylate de méthyle.**

**Stade A :**

(4S, 5S) 2,2-diméthyl 1,3-dioxolanne 4,5-diyl 3,3'-dipropénate de méthyle (Z).

On refroidit à −78°C sous atmosphère inerte 2,18 g de produit obtenu selon la préparation suivant le stade A de l'exemple 1, en solution dans 50 $cm^3$ de toluène, ajoute lentement 13,5 $cm^3$ d'une solution 1,5 M d'hydrure de diisobutylaluminium dans le toluène et maintient sous agitation à −78°C pendant 2 heures. On ajoute, goutte à goutte, à la solution obtenue, une solution de 8,35 g d'acétate de méthyltriphénylphosphoranilidène dans 150 $cm^3$ de méthanol et laisse le mélange réactionnel revenir à température ambiante. On agite 3 heures à 20°C, ajoute 50 $cm^3$ d'eau, évapore le méthanol sous pression réduite, extrait à l'éther, sèche et élimine les solvants sous pression réduite. On ajoute 250 $cm^3$ de pentane au résidu, filtre et évapore le filtrat à sec sous pression réduite. On obtient 2,5 g de produit brut que l'on chromatographie sur silice en éluant au mélange pentane-éther (7-3). on obtient 1,5 g de produit attendu. F = 51-52°C.

| | | |
|---|---|---|
| Analyse : | $C_{13}H_{18}O_6$ | |
| Calculé : | C%57,8 | H% 6,70 |
| Trouvé : | 57,95 | 6,70 |

**Stade B :**

[(4S, 5S) 2,2-diméthyl 1,3-dioxolanne 4,5-diyl] 3,3'-di-[2,2-diméthyl 1R, 3S cyclopropane carboxylate de méthyle].

On opère de manière analogue à celle décrite au stade A de l'exemple 2, en utilisant au départ 0,27 g de diester obtenu au stade précédent. On obtient 0,24 g de produit attendu. F = 97-98°C.

$[alpha]_D^{20}$ = −10,1° (c = 5,6 mg/ml $CHCl_3$).

Spectre de RMN ($CDCl_3$) :

δ= 0,9-1,6 (m avec 3s à 1,16 ; 1,25 et 1,36, 22H, $(CH_3)_2$ C et H cyclopropaniques),
3,56 (s, 6H, $CO_2CH_3$),
4,1-4,35 (m, 2H, CHO).

**Stade C :**

[(1S, 2S) 1,2-dihydroxyéthylène] 3,3'-di-[2,2-diméthyl 1R, 3S cyclopropane carboxylate de méthyle].

On opère de manière analogue à celle décrite au stade C de l'exemple 1, au départ des 0,24 g de produit obtenu au stade précédent. On obtient le produit attendu utilisé à l'état brut pour le stade suivant.

**Stade D :**

(1R, 3S) 3-formyl 2,2-diméthyl cyclopropane carboxylate de méthyle.

On opère de manière analogue à celle décrite au stade D de l'exemple 1, au départ de 0,31 g de produit obtenu comme décrit au stade précédent, en utilisant 0,32 g de périodate de sodium. On obtient 0,19 g de produit attendu.
$[alpha]_D^{20}$ = −73,2° (c = 15,5 mg/ml acétone).

## Revendications

**Revendications pour les Etats contractants suivants : AT, BE, CH, DE, FR, GB, IT, LI, LU, NL, SE**

1. Procédé de préparation des composés de formule (I) :

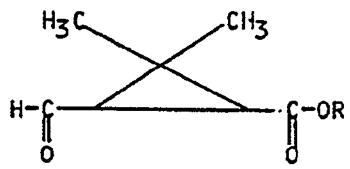

(I)

de structure cis ou trans, racémiques ou optiquement actifs, dans laquelle R représente un atome d'hydrogène, un radical alcoyle renfermant de 1 à 4 atomes de carbone ou un radical aryle renfermant jusqu'à 12 atomes de carbone, caractérisé en ce que l'on soumet à l'action d'un agent de réduction ménagée un diester de formule (II) :

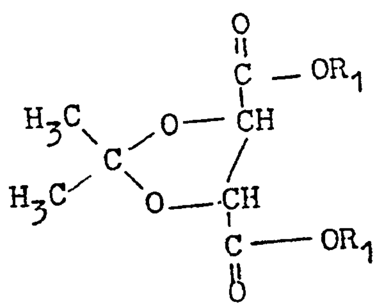

(II)

sous l'une quelconque de ses formes isomères dédoublées ou racémiques, dans laquelle $R_1$ représente un radical alcoyle renfermant de 1 à 4 atomes de carbone, un radical aryle renfermant jusqu'à 12 atomes de carbone ou un radical aralcoyle renfermant jusqu'à 8 atomes de carbone, pour obtenir un précurseur d'un dialdéhyde de formule (III) :

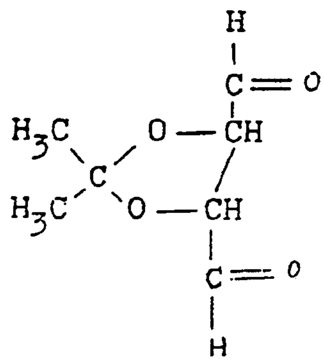

(III)

que l'on soumet à l'action d'un réactif de Wittig-Emmons-Horner de formule :

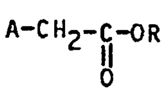

dans laquelle R est défini comme précédemment et A représente le reste organique phosphoré dudit réactif, pour obtenir le composé de formule (IV) :

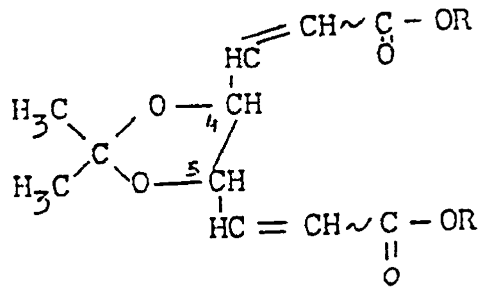

(IV)

dans laquelle R est défini comme précédemment et les traits ondulés schématisent une configuration cis ou trans, que l'on soumet à l'action d'un agent de gem-diméthyl cyclopropanation pour obtenir soit le composé de formule (V) :

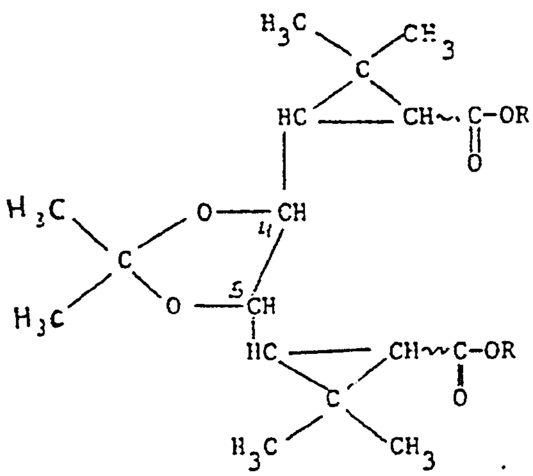

(V)

dans laquelle R et les traits ondulés sont définis comme précédemment, soit le composé de formule (Va) :

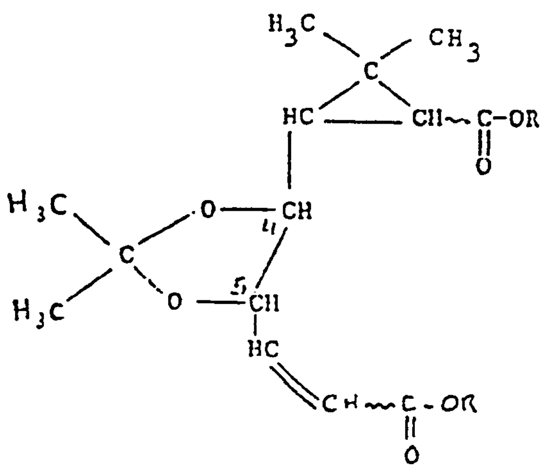

(Va)

dans laquelle R et les traits ondulés sont définis comme précédemment, composé de formule Va que, le cas échéant, l'on soumet à un agent de gem-diméthyl cyclopropanation, pour obtenir le composé de formule (V) définie précédemment, produit de formule (V) ou (Va) dont soit l'on hydrolyse le reste dioxolanne, pour obtenir respectivement ou bien le composé de formule (VI) :

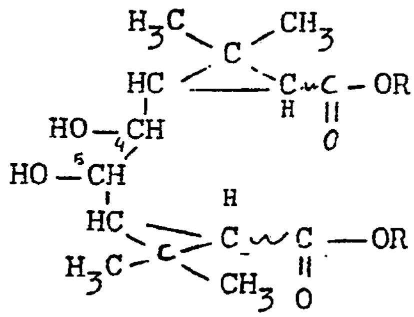

(VI)

dans laquelle R et les traits ondulés sont définis, comme précédemment, ou bien le composé de formule (VIa):

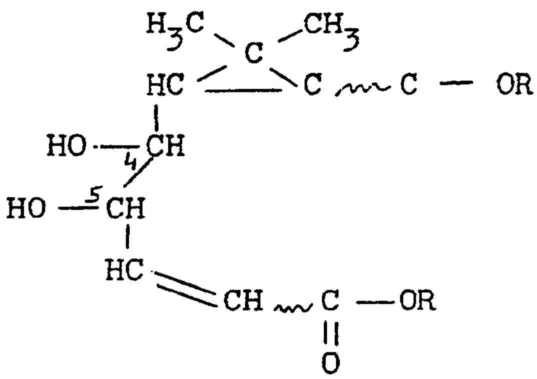

(VIa)

dans laquelle R et les traits ondulés sont définis comme précédemment, composé de formule (VI) ou (VIa), dont on clive la liaison 4, 5 pour obtenir le composé de formule (I) attendu, soit l'on hydrolyse le reste dioxolanne et clive simultanément la liaison 4, 5, pour obtenir le composé de formule (I) attendu.

2. Procédé selon la revendication 1, pour la préparation des composés de formule ($I_A$) :

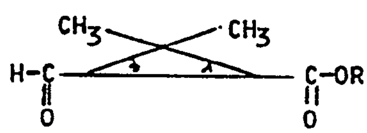

($I_A$)

de structure trans, racémiques ou optiquement actifs, dans laquelle R est défini comme précédemment, caractérisé en ce que l'on soumet à l'action d'un agent de réduction ménagée, un diester de formule (II), telle que définie précédemment, sous l'une quelconque de ses formes isomères dédoublées ou racémiques, pour obtenir un précurseur d'un dialdéhyde de formule (III) telle que définie précédemment, de structure isomérique correspondante, que l'on soumet à l'action d'un réactif de formule :

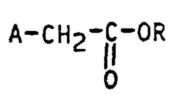

dans laquelle A et R sont définis comme précédemment, pour obtenir le composé de formule (IV') :

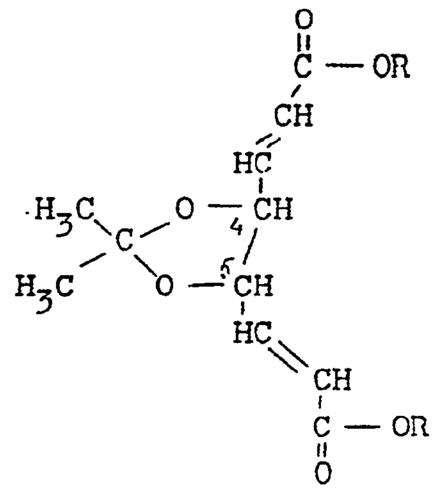

(IV')

dans laquelle R est défini comme précédemment et la géométrie des doubles liaisons est trans, que l'on soumet à l'action d'un agent de gem-diméthyl cyclopropanation pour obtenir le composé de formule (V') :

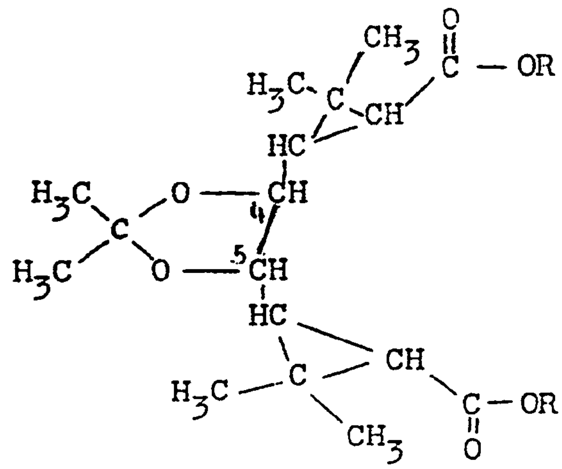

(V')

dans laquelle R est défini comme précédemment et la configuration des cycle cyclopropaniques est trans, dont soit l'on hydrolyse le reste dioxolanne pour obtenir le composé de formule (VI') :

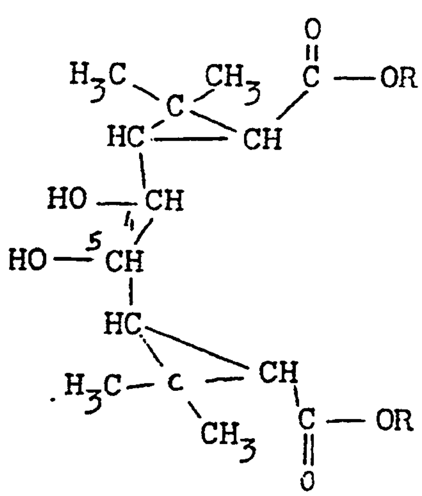

(VI')

dans laquelle R et la configuration des cycles cyclopropaniques sont définies comme précédemment, et clive

la liaison 4, 5, pour obtenir le composé de formule ($I_A$) correspondant, soit l'on hydrolyse le reste dioxolanne et clive simultanément la liaison 4, 5 pour obtenir le composé de formule ($I_A$) attendu.

3. Procédé selon la revendication 2, pour la préparation des composés de formule ($I_A$) de configuration 1R trans, caractérisé en ce que l'on soumet à l'action de l'hydrure de diisobutylaluminium un diester de formule ($II_1$) :

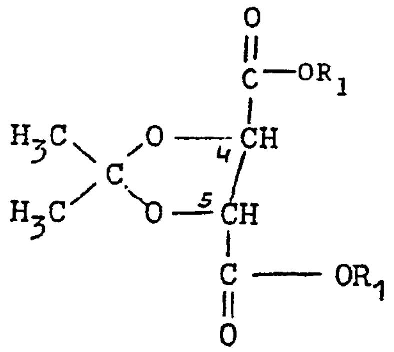

($II_1$)

de configuration (4R, 5R), dans laquelle $R_1$ est défini comme précédemment, pour obtenir le précurseur correspondant du dialdéhyde de formule ($III_1$) :

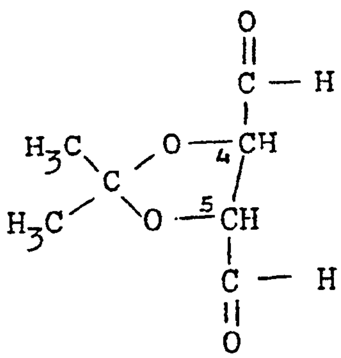

($III_1$)

de configuration (4R, 5R), que l'on soumet à l'action d'un réactif de formule :

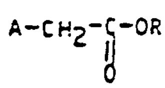

dans laquelle A et R sont définis comme précédemment, pour obtenir le composé de formule ($IV'_1$) :

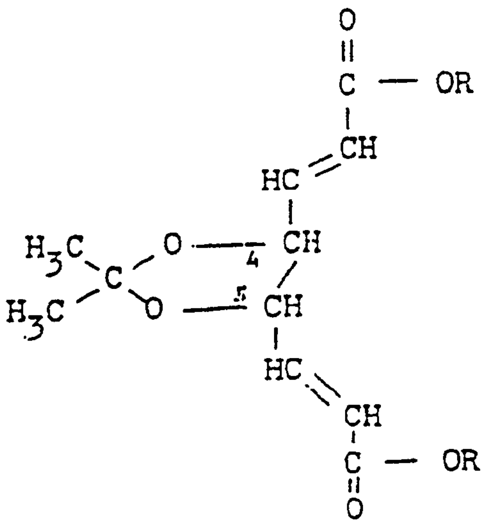

(IV'$_1$)

de configuration (4S, 5S), dans laquelle R est défini comme précédemment et la géométrie des doubles liaisons est trans, que l'on soumet à l'action d'un isopropylidène triphénylphosphorane pour obtenir le composé de formule (V'$_1$) :

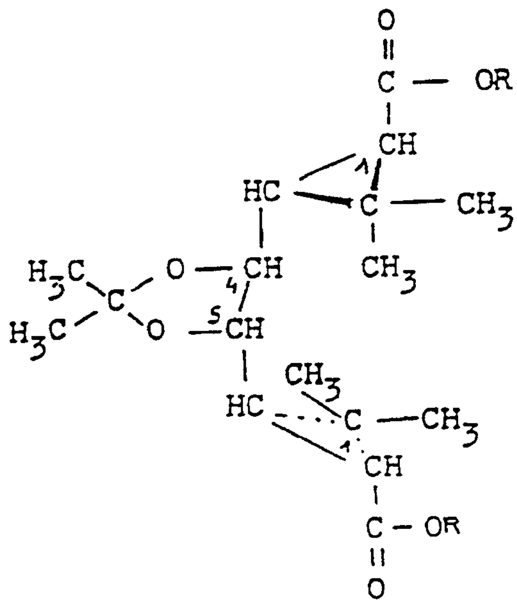

(V'$_1$)

de configuration (4S, 5S) et (1R trans) au niveau des cycles cylopropanes, dans laquelle R est défini comme précédemment, dont soit l'on hydrolyse le reste dioxolanne, pour obtenir le composé de formule (VI'$_1$) :

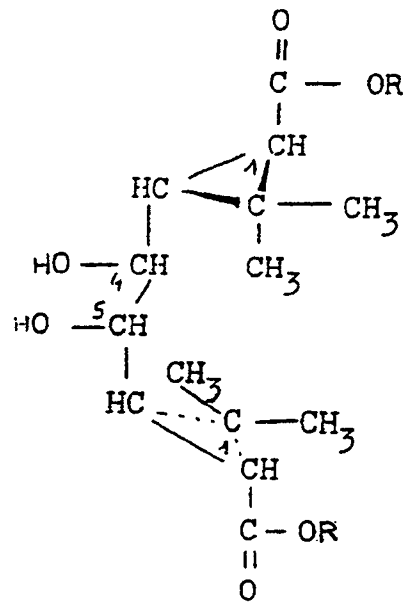

(VI'$_1$)

de configuration (4S, 5S) et (1R trans) au niveau des cycles cyclopropanes, dans laquelle R est défini comme précédemment et clive la liaison 4, 5 par action d'un agent oxydant, pour obtenir le composé de formule ($I_A$) de configuration (1R trans), soit l'on hydrolyse le reste dioxolanne et clive simultanément la liaison 4, 5 pour obtenir le composé de formule ($I_A$) de configuration (1R trans).

4. Procédé selon la revendication 1, pour la préparation des composés de formule ($I_B$) :

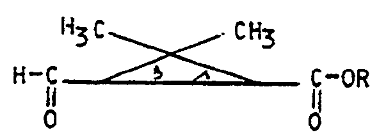

($I_B$)

de structure cis, racémiques ou optiquement actifs, dans laquelle R est défini comme précédemment, caractérisé en ce que l'on soumet à l'action d'un agent de réduction ménagée, un diester de formule (II), telle que définie précédemment, sous l'une quelconque de ses formes isomères dédoublées ou racémiques, pour obtenir un précurseur d'un dialdéhyde de formule (III) telle que définie précédemment, de structure isomérique correspondante, que l'on soumet à l'action d'un réactif de formule :

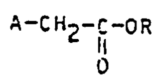

dans laquelle A et R sont définis comme précédemment, pour obtenir le composé de formule (IV") :

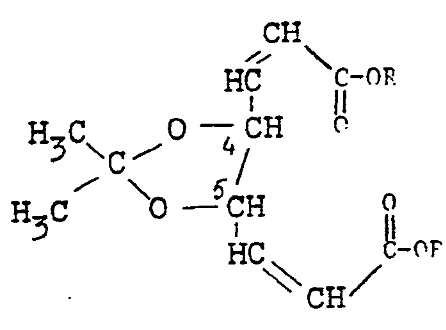

(IV")

dans laquelle R est défini comme précédemment et la géométrie des doubles liaisons est cis, que l'on soumet

à l'action d'un isopropylidène diphényl sulfurane pour obtenir le composé de formule (V") :

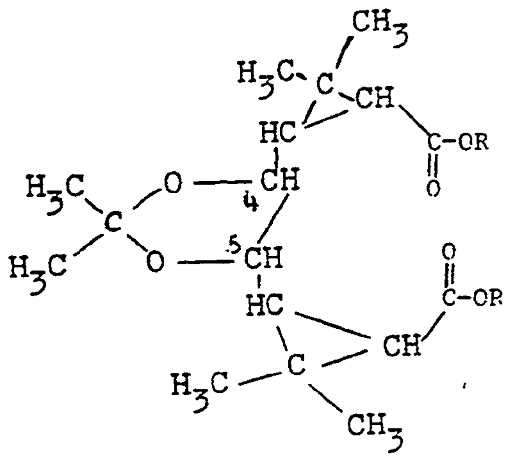

(V")

dans laquelle R est défini comme précédemment et la configuration des cycle cyclopropaniques est cis, dont soit l'on hydrolyse le reste dioxolanne pour obtenir le composé de formule (VI") :

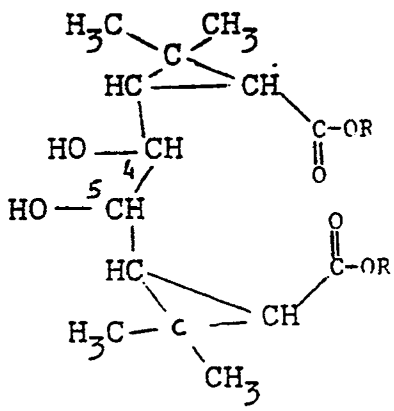

(VI")

dans laquelle R et la configuration des cycles cyclopropaniques sont définies comme précédemment, et clive la liaison 4, 5, pour obtenir le composé de formule ($I_B$) correspondant, soit l'on hydrolyse le reste dioxolanne et clive simultanément la liaison 4, 5 pour obtenir le composé de formule ($I_B$) attendu.

5. Procédé selon la revendication 4, pour la préparation des composés de formule ($I_B$) de configuration 1R cis, caractérisé en ce que l'on soumet à l'action de l'hydrure de diisobutylaluminium un diester de formule ($II_1$):

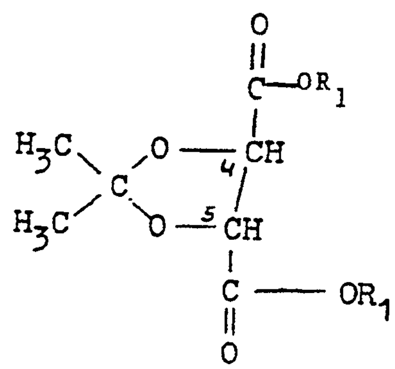

($II_1$)

de configuration (4R, 5R), dans laquelle $R_1$ est défini comme précédemment, pour obtenir le précurseur correspondant du dialdéhyde de formule ($III_1$) :

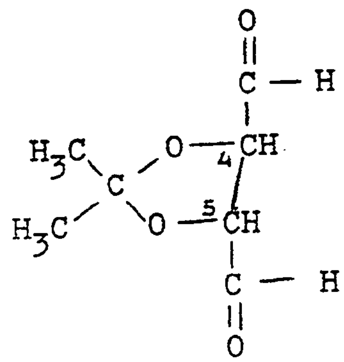

$(III_1)$

de configuration (4R, 5R), que l'on soumet à l'action d'un réactif de formule :

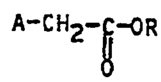

dans laquelle A et R sont définis comme précédemment, pour obtenir le composé de formule $(IV''_1)$ :

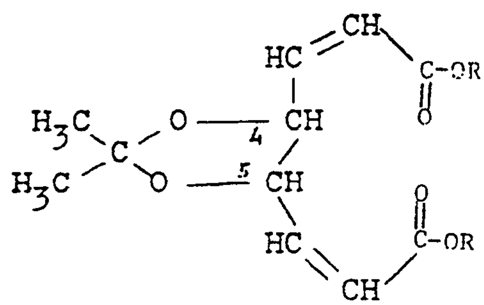

$(IV''_1)$

de configuration (4S, 5S), dans laquelle R est défini comme précédemment et la géométrie des doubles liaisons est cis, que l'on soumet à l'action d'un isopropylidène diphénylsulfurane pour obtenir le composé de formule $(V''_1)$ :

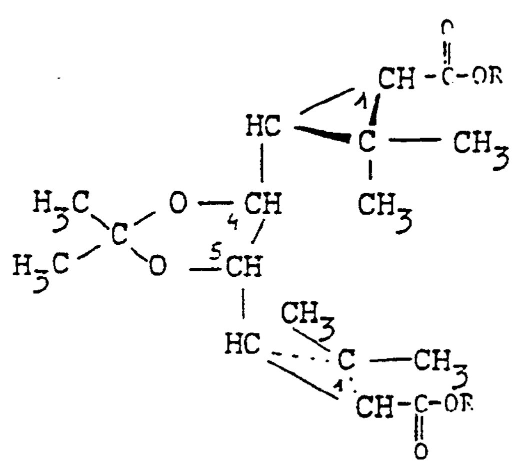

$(V''_1)$

de configuration (4S, 5S) et (1R cis) au niveau des cycles cylopropanes, dans laquelle R est défini comme précédemment, dont soit l'on hydrolyse le reste dioxolanne, pour obtenir le composé de formule $(VI''_1)$ :

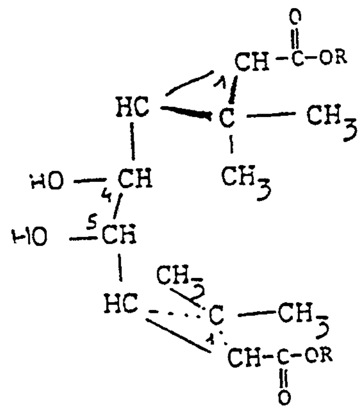

(VI"$_1$)

de configuration (4S, 5S) et (1R cis) au niveau des cycles cyclopropanes, dans laquelle R est défini comme précédemment et clive la liaison 4, 5 par action d'un agent oxydant, pour obtenir le composé de formule ($I_B$) de configuration (1R cis), soit l'on hydrolyse le reste dioxolanne et clive simultanément la liaison 4, 5 pour obtenir le composé de formule ($I_B$) de configuration (1R cis).

6. Les composés de formules (IV), (V), (Va), (VI) et (VIa) telles que définies à la revendication 1.

7. Le composé tel qu'obtenu par la réduction du diester de formule ($II_1$) ; telle que définie à la revendication 3, par l'hydrure de diisobutylaluminium.

**Revendications pour l'Etat contractant suivant : ES**

1. Procédé de préparation des composés de formule (I) :

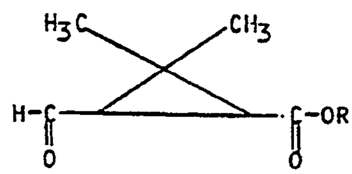

(I)

de structure cis ou trans, racémiques ou optiquement actifs, dans laquelle R représente un atome d'hydrogène, un radical alcoyle renfermant de 1 à 4 atomes de carbone ou un radical aryle renfermant jusqu'à 12 atomes de carbone, caractérisé en ce que l'on soumet à l'action d'un agent de réduction ménagée un diester de formule (II) :

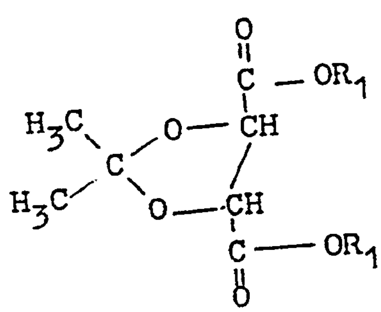

(II)

sous l'une quelconque de ses formes isomères dédoublées ou racémiques, dans laquelle $R_1$ représente un radical alcoyle renfermant de 1 à 4 atomes de carbone, un radical aryle renfermant jusqu'à 12 atomes de carbone ou un radical aralcoyle renfermant jusqu'à 8 atomes de carbone, pour obtenir un précurseur d'un dialdéhyde de formule (III) :

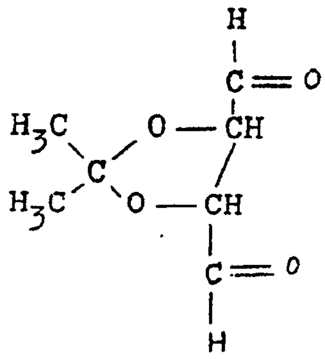

(III)

que l'on soumet à l'action d'un réactif de Wittig-Emmons-Horner de formule :

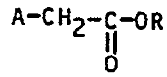

dans laquelle R est défini comme précédemment et A représente le reste organique phosphoré dudit réactif, pour obtenir le composé de formule (IV) :

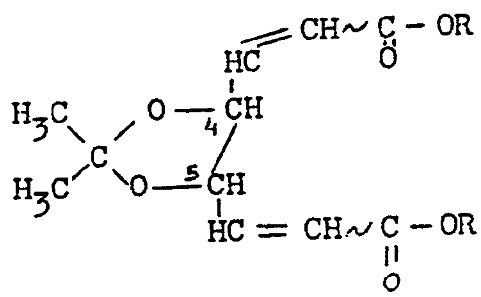

(IV)

dans laquelle R est défini comme précédemment et les traits ondulés schématisent une configuration cis ou trans, que l'on soumet à l'action d'un agent de gem-diméthyl cyclopropanation pour obtenir soit le composé de formule (V) :

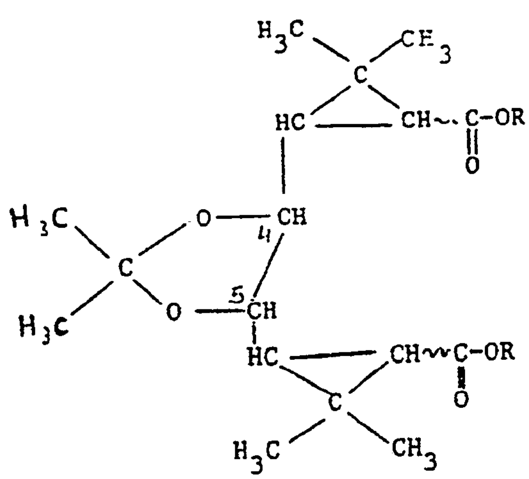

(V)

dans laquelle R et les traits ondulés sont définis comme précédemment, soit le composé de formule (Va) :

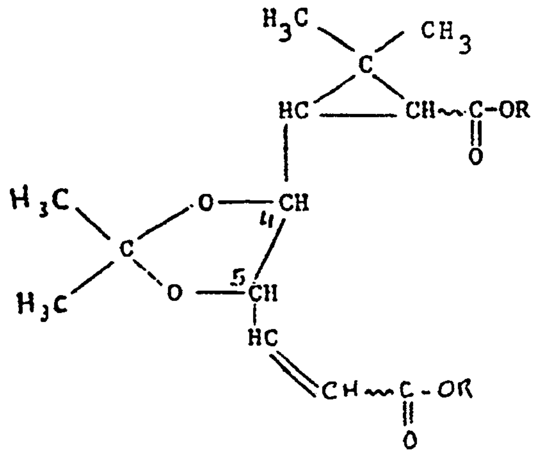

(Va)

dans laquelle R et les traits ondulés sont définis comme précédemment, composé de formule Va que, le cas échéant, l'on soumet à un agent de gem-diméthyl cyclopropanation, pour obtenir le composé de formule (V) définie précédemment, produit de formule (V) ou (Va) dont soit l'on hydrolyse le reste dioxolanne, pour obtenir respectivement ou bien le composé de formule (VI) :

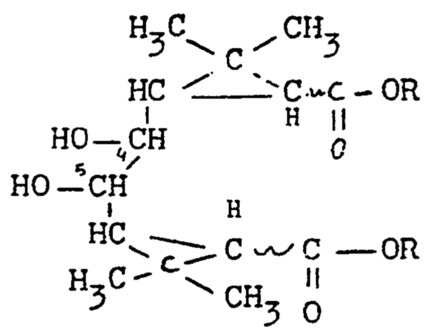

(VI)

dans laquelle R et les traits ondulés sont définis, comme précédemment, ou bien le composé de formule (VIa):

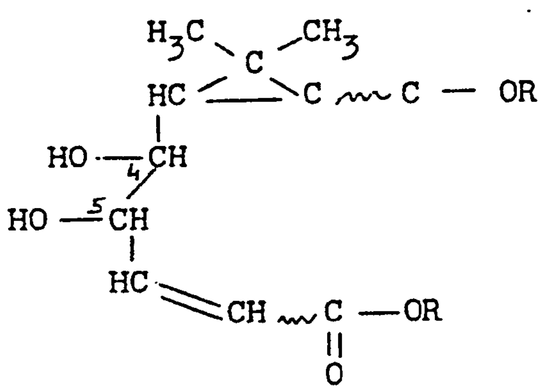

(VIa)

dans laquelle R et les traits ondulés sont définis comme précédemment, composé de formule (VI) ou (VIa), dont on clive la liaison 4, 5 pour obtenir le composé de formule (I) attendu, soit l'on hydrolyse le reste dioxolanne et clive simultanément la liaison 4, 5, pour obtenir le composé de formule (I) attendu.

2. Procédé selon la revendication 1, pour la préparation des composés de formule ($I_A$) :

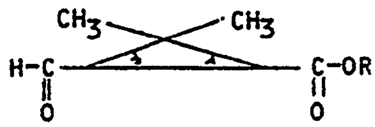

$(I_A)$

de structure trans, racémiques ou optiquement actifs, dans laquelle R est défini comme précédemment, caractérisé en ce que l'on soumet à l'action d'un agent de réduction ménagée, un diester de formule (II), telle que définie précédemment, sous l'une quelconque de ses formes isomères dédoublées ou racémiques, pour obtenir un précurseur d'un dialdéhyde de formule (III) telle que définie précédemment, de structure isomérique correspondante, que l'on soumet à l'action d'un réactif de formule :

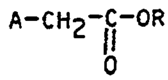

dans laquelle A et R sont définis comme précédemment, pour obtenir le composé de formule (IV') :

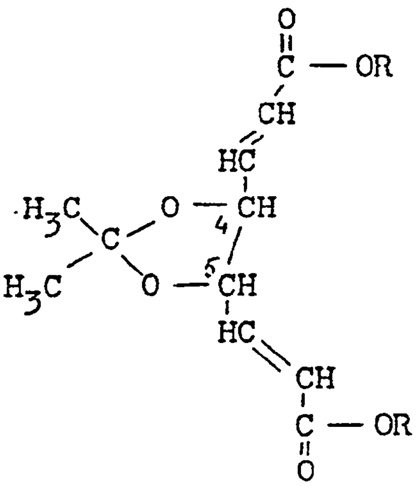

(IV')

dans laquelle R est défini comme précédemment et la géométrie des doubles liaisons est trans, que l'on soumet à l'action d'un agent de gem-diméthyl cyclopropanation pour obtenir le composé de formule (V') :

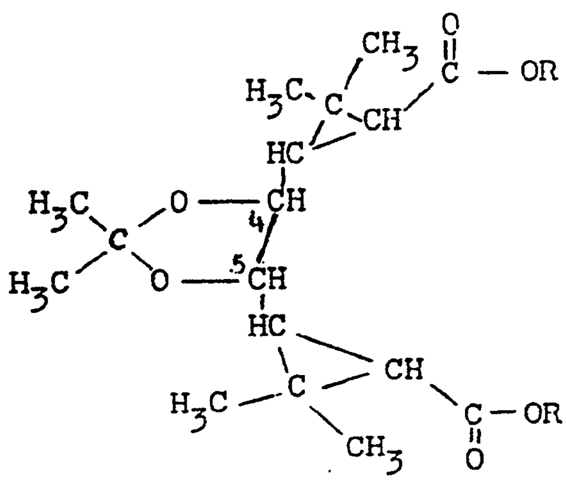

(V')

dans laquelle R est défini comme précédemment et la configuration des cycle cyclopropaniques est trans, dont soit l'on hydrolyse le reste dioxolanne pour obtenir le composé de formule (VI') :

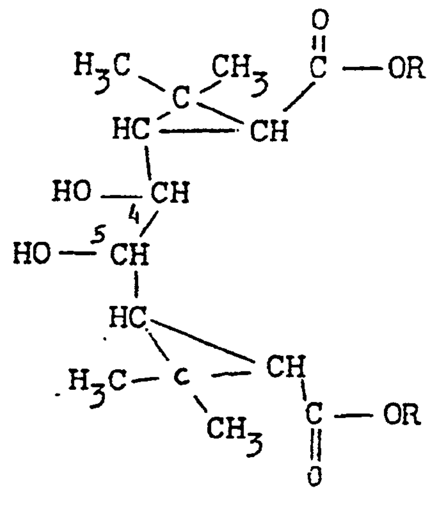

(VI')

dans laquelle R et la configuration des cycles cyclopropaniques sont définies comme précédemment, et clive la liaison 4, 5, pour obtenir le composé de formule ($I_A$) correspondant, soit l'on hydrolyse le reste dioxolanne et clive simultanément la liaison 4, 5 pour obtenir le composé de formule ($I_A$) attendu.

3. Procédé selon la revendication 2, pour la préparation des composés de formule ($I_A$) de configuration 1R trans, caractérisé en ce que l'on soumet à l'action de l'hydrure de diisobutylaluminium un diester de formule ($II_1$) :

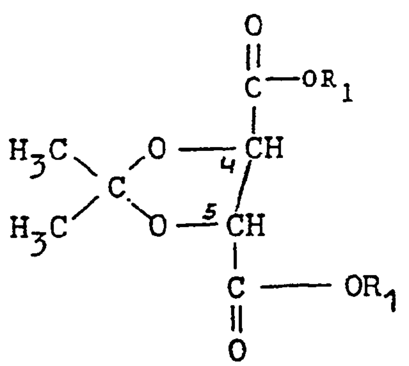

($II_1$)

de configuration (4R, 5R), dans laquelle $R_1$ est défini comme précédemment, pour obtenir le précurseur correspondant du dialdéhyde de formule ($III_1$) :

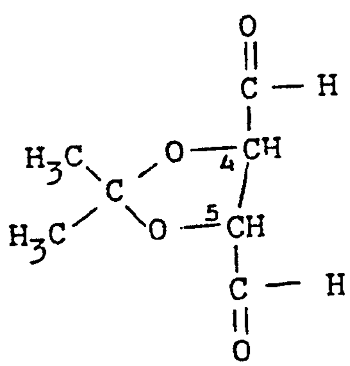

($III_1$)

de configuration (4R, 5R), que l'on soumet à l'action d'un réactif de formule :

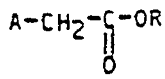

dans laquelle A et R sont définis comme précédemment, pour obtenir le composé de formule (IV'$_1$) :

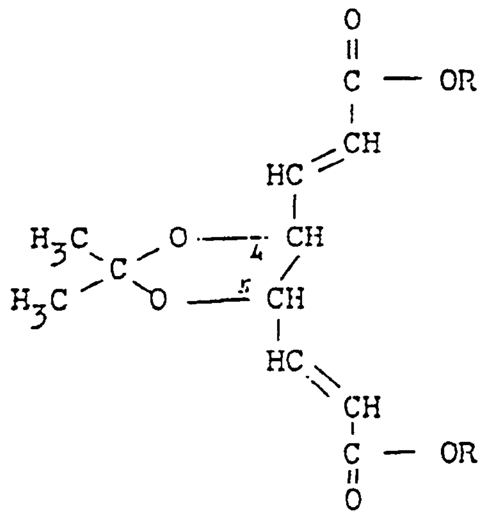

(IV'$_1$)

de configuration (4S, 5S), dans laquelle R est défini comme précédemment et la géométrie des doubles liaisons est trans, que l'on soumet à l'action d'un isopropylidène triphénylphosphorane pour obtenir le composé de formule (V'$_1$) :

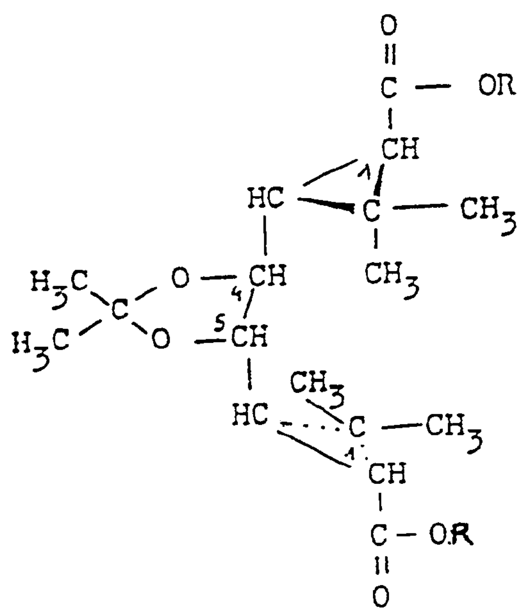

(V'$_1$)

de configuration (4S, 5S) et (1R trans) au niveau des cycles cylopropanes, dans laquelle R est défini comme précédemment, dont soit l'on hydrolyse le reste dioxolanne, pour obtenir le composé de formule (VI'$_1$) :

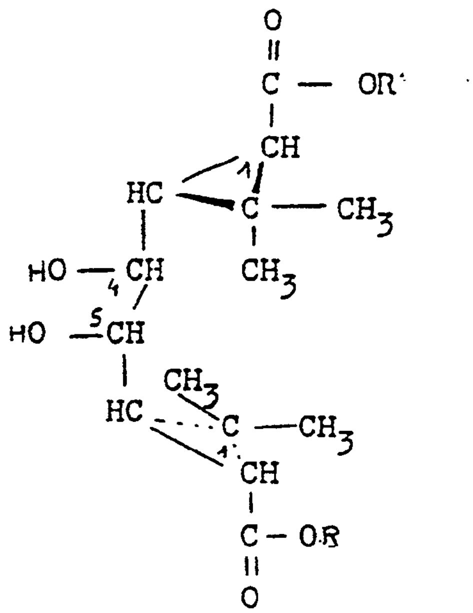

(VI'$_1$)

de configuration (4S, 5S) et (1R trans) au niveau des cycles cyclopropanes, dans laquelle R est défini comme précédemment et clive la liaison 4, 5 par action d'un agent oxydant, pour obtenir le composé de formule ($I_A$) de configuration (1R trans), <u>soit</u> l'on hydrolyse le reste dioxolanne et clive simultanément la liaison 4, 5 pour obtenir le composé de formule ($I_A$) de configuration (1R trans).

4. Procédé selon la revendication 1, pour la préparation des composés de formule ($I_B$) :

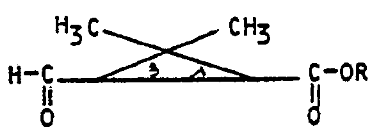

($I_B$)

de structure cis, racémiques ou optiquement actifs, dans laquelle R est défini comme précédemment, caractérisé en ce que l'on soumet à l'action d'un agent de réduction ménagée, un diester de formule (II), telle que définie précédemment, sous l'une quelconque de ses formes isomères dédoublées ou racémiques, pour obtenir un précurseur d'un dialdéhyde de formule (III) telle que définie précédemment, de structure isomérique correspondante, que l'on soumet à l'action d'un réactif formule :

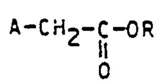

dans laquelle A et R sont définis comme précédemment, pour obtenir le composé de formule (IV") :

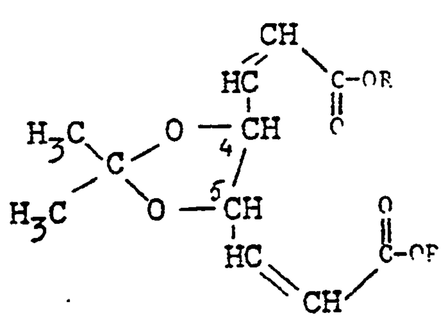

(IV")

dans laquelle R est défini comme précédemment et la géométrie des doubles liaisons est cis, que l'on soumet à l'action d'un isopropylidène diphényl sulfurane pour obtenir le composé de formule (V") :

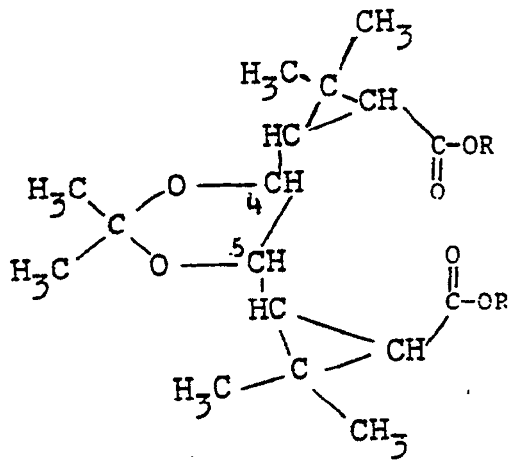

(V")

dans laquelle R est défini comme précédemment et la configuration des cycle cyclopropaniques est cis, dont soit l'on hydrolyse le reste dioxolanne pour obtenir le composé de formule (VI") :

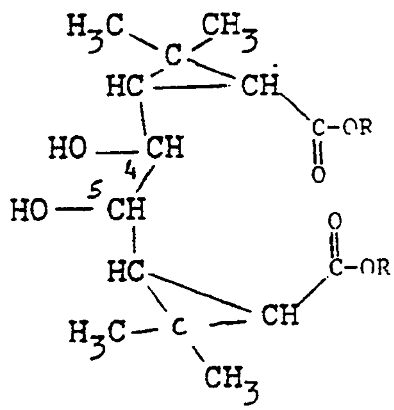

(VI")

dans laquelle R et la configuration des cycles cyclopropaniques sont définies comme précédemment, et clive la liaison 4, 5, pour obtenir le composé de formule ($I_B$) correspondant, soit l'on hydrolyse le reste dioxolanne et clive simultanément la liaison 4, 5 pour obtenir le composé de formule ($I_B$) attendu.

5. Procédé selon la revendication 4, pour la préparation des composés de formule ($I_B$) de configuration 1R cis, caractérisé en ce que l'on soumet à l'action de l'hydrure de diisobutylaluminium un diester de formule ($II_1$):

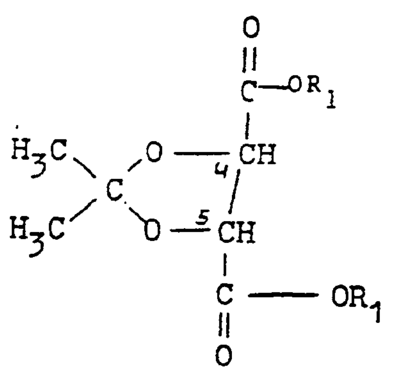

($II_1$)

de configuration (4R, 5R), dans laquelle $R_1$ est défini comme précédemment, pour obtenir le précurseur correspondant du dialdéhyde de formule ($III_1$) :

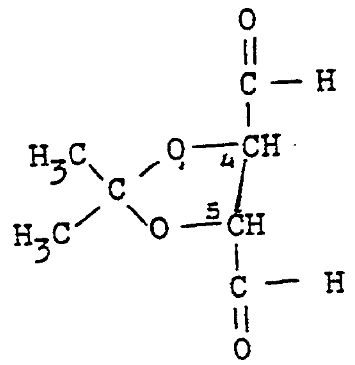

(III$_1$)

de configuration (4R, 5R), que l'on soumet à l'action d'un réactif de formule :

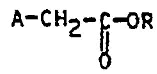

dans laquelle A et R sont définis comme précédemment, pour obtenir le composé de formule (IV"$_1$) :

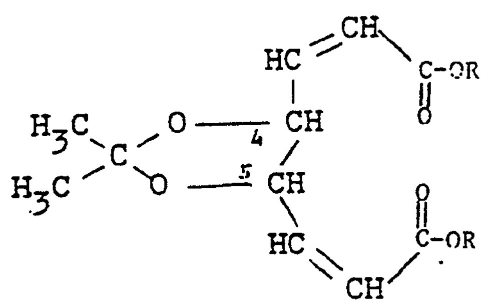

(IV"$_1$)

de configuration (4S, 5S), dans laquelle R est défini comme précédemment et la géométrie des doubles liaisons est cis, que l'on soumet à l'action d'un isopropylidène diphénylsulfurane pour obtenir le composé de formule (V"$_1$) :

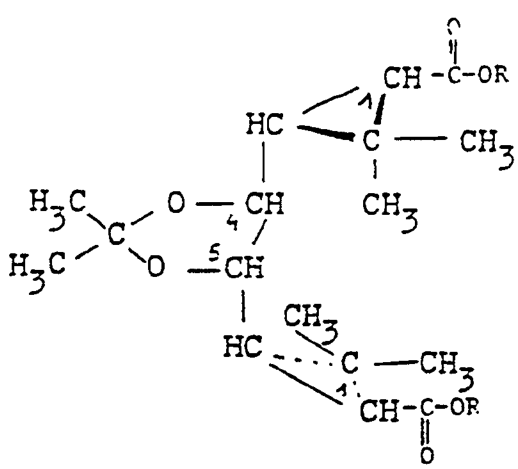

(V"$_1$)

de configuration (4S, 5S) et (1R cis) au niveau des cycles cylopropanes, dans laquelle R est défini comme précédemment, dont soit l'on hydrolyse le reste dioxolanne, pour obtenir le composé de formule (VI"$_1$) :

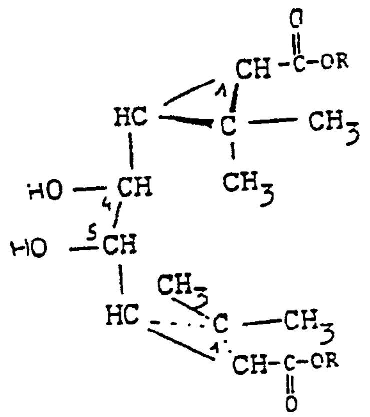

(VI"$_1$)

de configuration (4S, 5S) et (1R cis) au niveau des cycles cyclopropanes, dans laquelle R est défini comme précédemment et clive la liaison 4, 5 par action d'un agent oxydant, pour obtenir le composé de formule ($I_B$) de configuration (1R cis), soit l'on hydrolyse le reste dioxolanne et clive simultanément la liaison 4, 5 pour obtenir le composé de formule ($I_B$) de configuration (1R cis).

## Patentansprüche

### Patentansprüche für folgende Vertragsstaaten : AT, BE, CH, DE, FR, GB, IT, LI, LU, NL, SE

1. Verfahren zur Herstellung von Verbindungen der Formel (I) :

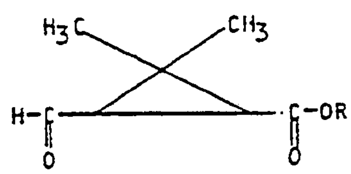

(I)

mit cis- oder trans-Struktur in racemischer oder optisch aktiver Form, worin R für ein Wasserstoffatom, einen Alkylrest mit 1 bis 4 Kohlenstoffatomen oder einen Arylrest mit bis zu 12 Kohlenstoffatomen steht, dadurch gekennzeichnet, daß man einen Diester der Formel (II) :

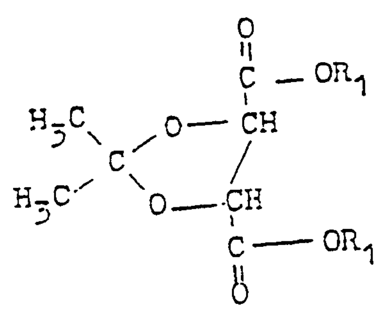

(II)

in irgendeiner seiner getrennten oder racemischen isomeren Formen, worin $R_1$ für einen Alkylrest mit 1 bis 4 Kohlenstoffatomen, einen Arylrest mit bis zu 12 Kohlenstoffatomen oder einen Aralkylrest mit bis zu 8 Kohlenstoffatomen steht, der Einwirkung eines Mittels für eine schonende Reduktion unterzieht, um zu einem Vorläufer eines Dialdehyds der Formel (III) :

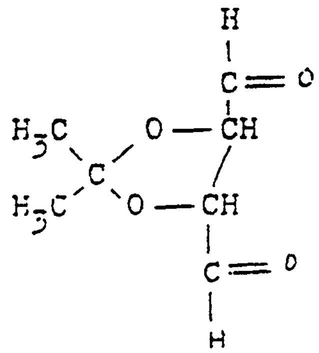

(III)

zu gelangen, welchen man der Einwirkung eines Wittig-Emmons-Horner-Reagens der Formel

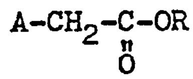

worin R wie vorstehend definiert ist und A den organicschen Phosphorrest dieses Reagens wiedergibt, unterzieht, um zu der Verbindung der Formel (IV)

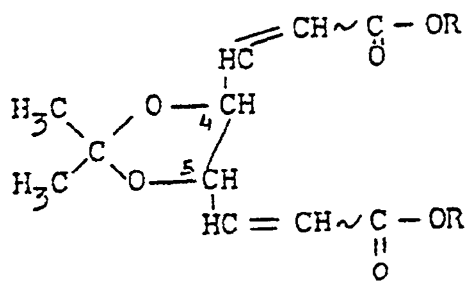

(IV)

zu gelangen, worin R wie vorstehend defiliert ist und die gewellten Linien eine cis- oder trans-Konfiguration schematisieren, welche man der Einwirkung eines Mittels für die gem-Dimethylcyclopropanierung unterzieht, um entweder zu der Verbindung der Formel (V)

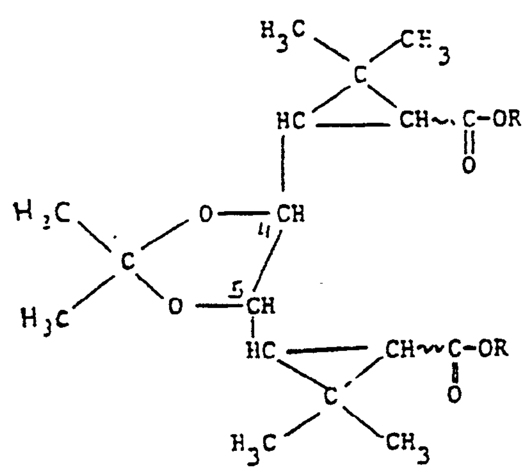

(V)

worin R und die gewellten Linien wie vorstehend definiert sind, oder zu der Verbindung der Formel (Va)

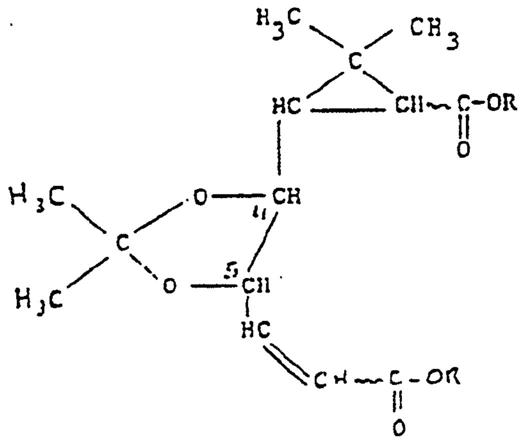

(Va)

zu gelangen, worin R und die gewellten Linien wie vorstehend definiert sind, welche Verbindung der Formel Va man gegebenenfalls der Einwirkung eines Mittels für die gem-Dimethylcyclopropanierung unterzieht, um zu der Verbindung der vorstehend definierten Formel (V) zu gelangen, man entweder den Dioxolanrest des Produkts der Formel (V) oder (Va) hydrolysiert, um entweder die Verbindung der Formel (VI)

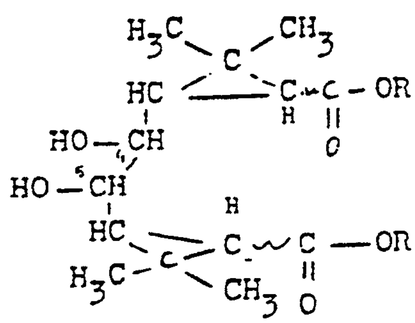

(VI)

worin R und die gewellten Linien wie vorstehend definiert sind, oder die Verbindung der Formel (VIa)

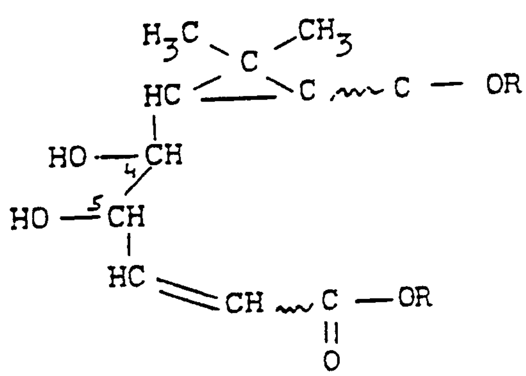

(VIa)

worin R und die gewellten Linien wie vorstehend definiert sind, zu erhalten, und die 4,5-Bindung der Verbindung der Formel (VI) oder (VIa) spaltet, um zu der erwarteten Verbindung der Formel (I) zu gelangen, oder den Dioxolanrest hydrolysiert und gleichzeitig die 4,5-Bindung spaltet, um die erwartete Verbindung der Formel (I) zu erhalten.

2. Verfahren gemäß Anspruch 1 zur Herstellung der Verbindungen der Formel ($I_A$)

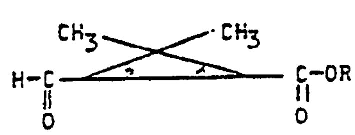

($I_A$)

mit racemischer oder optisch aktiver trans-Struktur, worin R wie vorstehend definiert ist, dadurch gekennzeichnet, daß man einen Diester der Formel (II), wie vorstehend definiert, in irgendeiner seiner getrennten oder racemischen isomeren Formen der Einwirkung eines Mittels zur schonenden Reduktion unterzieht, um zu einem Vorläufer für einen Dialdehyd der Formel (III), wie vorstehend definiert, mit der entsprechenden isomeren Struktur zu gelangen, welchen man der Einwirkung eines Reagens der Formel

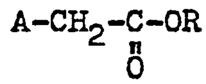

unterzieht, worin A und R wie vorstehend definiert sind, um zu der Verbindung der Formel (IV')

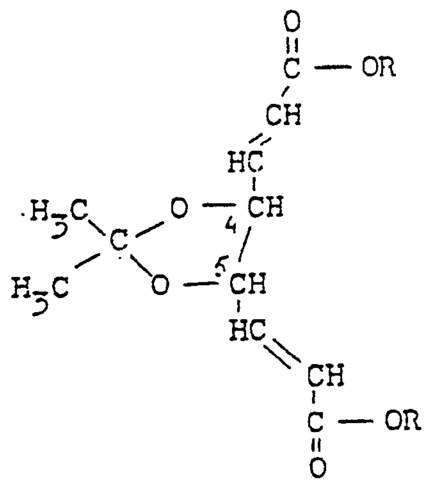

(IV')

zu gelangen, worin R wie vorstehend definiert ist und die Geometrie der Doppelbindungen die trans-Geometrie ist, welche man der Einwirkung eines Mittels zur gem-Dimethylcyclopropanierung unterzieht, um zu der Verbindung der Formel (V')

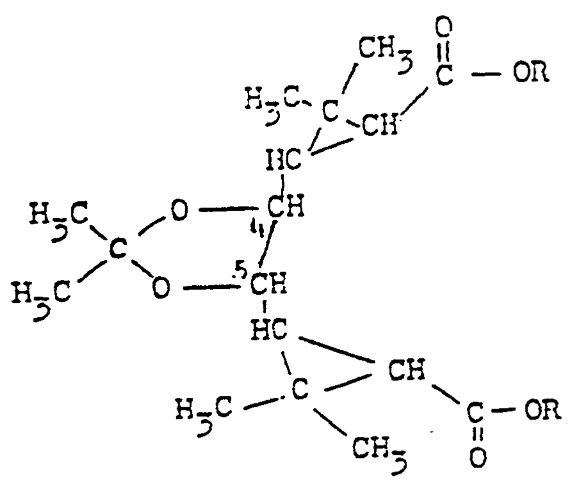

(V')

zu gelangen, worin R wie vorstehend definiert ist und die Konfiguration der Cyclopropanringe trans-ständig ist, man <u>entweder</u> deren Dioxolanrest hydrolysiert, um die Verbindung der Formel (VI')

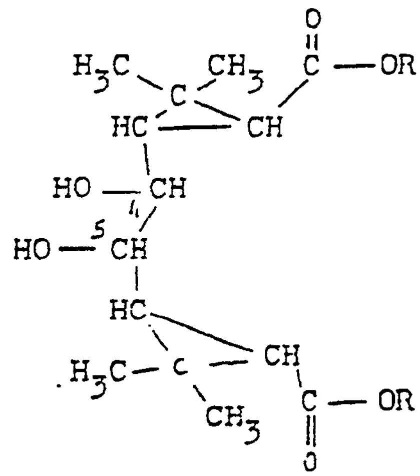

(VI')

zu erhalten, worin R und die Konfiguration der Cyclopropanringe wie vorstehend definiert sind, und die 4,5-Bindung spaltet, um die entsprechende Verbindung der Formel ($I_A$) zu erhalten, oder den Dioxolanrest hydrolysiert und gleichzeitig die 4,5-Bindung spaltet, um die erwartete Verbindung der Formel ($I_A$) zu erhalten.

3. Verfahren gemäß Anspruch 2 zur Herstellung der Verbindungen der Formel ($I_A$) mit 1R-trans-Konfiguration, dadurch gekennzeichnet, daß man eien Diester der Formel $(II_1)$

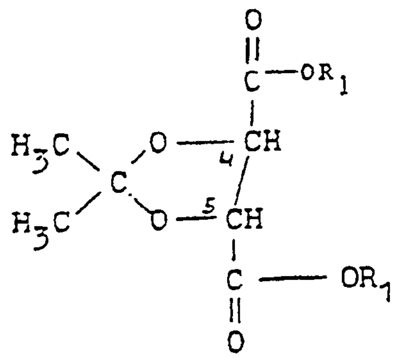

$(II_1)$

mit (4R, 5R)-Konfiguration, worin $R_1$ wie vorstehend defiliert ist, der Einwirkung von Diisobutylaluminiumhydrid unterzieht, um zu dem entsprechenden Vorläufer des Dialdehyds der Formel ($III_1$)

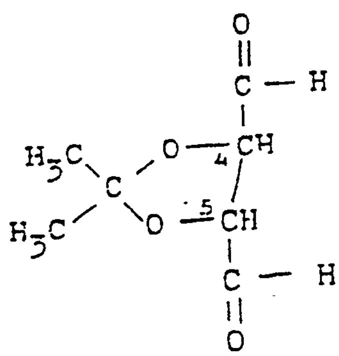

($III_1$)

mit (4R, 5R)-Konfiguration zu gelangen, welchen man der Einwirkung eines Reagens der Formel

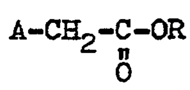

unterzieht, worin A und R wie vorstehend definiert sind, um zu der Verbindung der Formel (IV'$_1$)

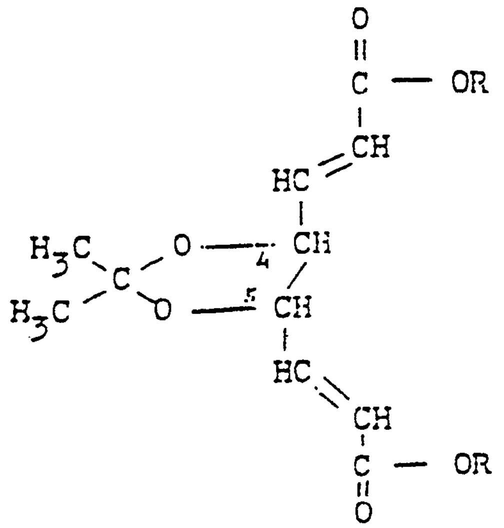

(IV'$_1$)

mit (4S, 5S)-Konfiguration zu gelangen, worin R wie vorstehend definiert ist und die Geometrie der Doppelbindungen die trans-Geometrie ist, welche man der Einwirkung eines Isopropylidentriphenylphosphorans unterzieht, um zu der Verbindung der Formel (V'$_1$)

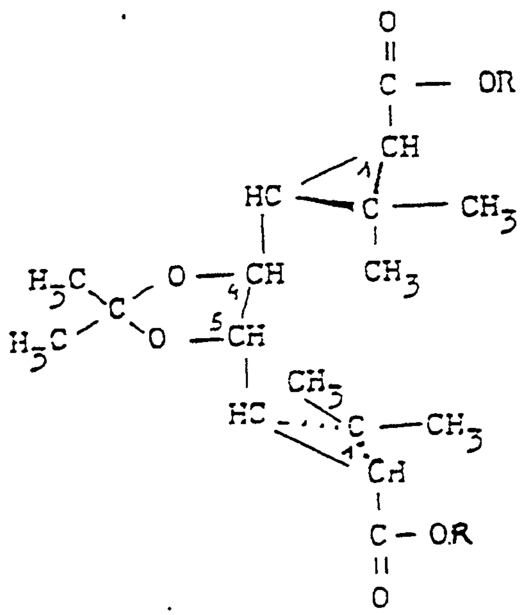

(V'$_1$)

mit (4S, 5S)- und (1R-trans)-Konfiguration hinsichtlich der Cyclopropanringe zu gelangen, worin R wie vorstehend definiert ist, man entweder deren Dioxolanrest hydrolysiert, um zu der Verbindung der Formel (VI'$_1$)

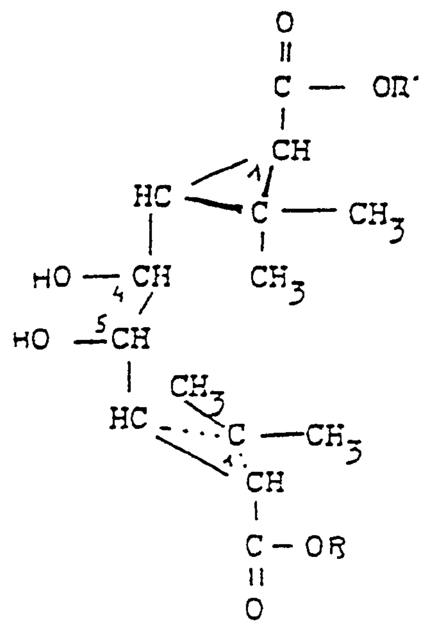

(VI'$_1$)

mit (4S, 5S)- und (1R-trans)-Konfiguration hinsichtlich der Cyclopropanringe zu gelangen, worin R wie vorstehend definiert ist, und die 4,5-Bindung durch ein Oxidationsmittel spaltet, um die Verbindung der Formel ($I_A$) mit (1R-trans)-Konfiguration zu erhalten, oder den Dioxolanrest hydrolysiert und gleichzeitig die 4,5-Bindung spaltet, um die Verbindung der Formel ($I_A$) mit (1R-trans)-Konfiguration zu erahlten.

4. Verfahren gemäß Anspruch 1 zur Herstellung von Verbindungen der Formel ($I_B$)

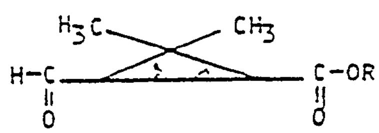

($I_B$)

mit racemischer oder optisch aktiver cis-Struktur, worin R wie vorstehend definiert ist, dadurch gekennzeichnet, daß man einen Diester der Formel (II), wie vorstehend definiert, in irgendeiner seiner getrennten oder racemischen isomeren Formen der Einwirkung eines Mittels für die schonende Reduktion unterzieht, um zu einem Vorläufer eines Dialdehyds der Formel (III), wie vorstehend definiert, mit entsprechender isomerer Struktur zu gelangen, welchen man der Einwirkung eines Reagens der Formel

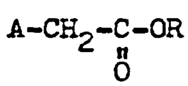

unterzieht, worin A und R wie vorstehend definiert sind, um zu der Verbindung der Formel (IV")

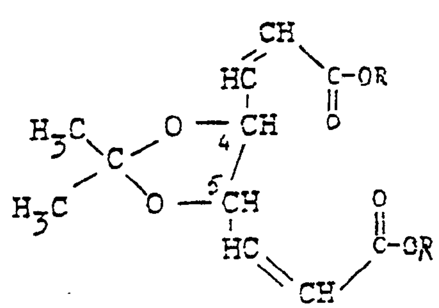

(IV")

zu gelangen, worin R wie vorstehend definiert ist und die Geometrie der Doppelbindungen die cis-Geometrie ist, welche man der Einwirkung eines Isopropylidendiphenylsulfurans unterzieht, um die Verbindung der Formel

(V")

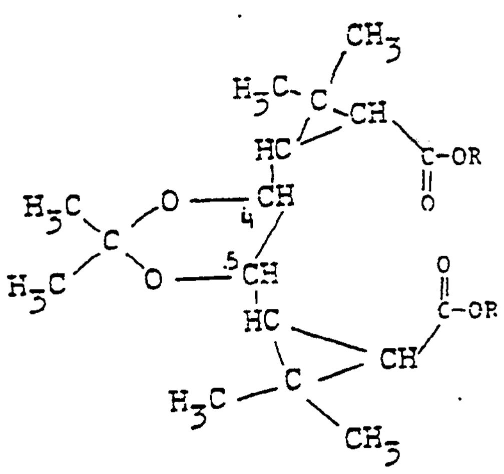

(V")

zu erhalten, worin R wie vorstehend definiert ist und die Konfiguration der Cyclopropanringe die cis-Konfiguration ist, man entweder deren Dioxolanrest hydrolysiert, um die Verbindung der Formel (VI")

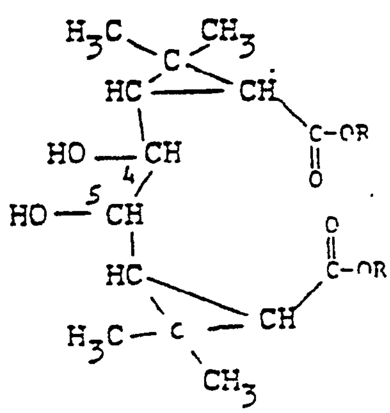

(VI")

zu erhalten, worin R und die Konfiguration der Cyclopropanringe wie vorstehend definiert sind, und die 4,5-Bindung spaltet, um die entsprechende Verbindung der Formel ($I_B$) zu erhalten, oder den Dioxolanrest hydrolysiert und gleichzeitig die 4,5-Bindung spaltet, um die erwartete Verbidung der Formel ($I_B$) zu erhalten.

5. Verfharen gemäß Anspruch 4 zur Herstellung von Verbidungen der Formel ($I_B$) mit 1R-cis-Konfiguration, dadurch gekennzeichnet, daß man einen Diester der Formel ($II_1$)

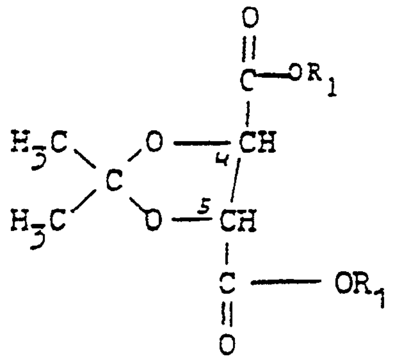

(II$_1$)

mit (4R, 5R)-Konfiguration, worin $R_1$ wie vorstehend definiert ist, der Einwirkung von Diisobutylaluminiumhydrid unterzieht, um zu dem entsprechenden Vorläufer des Dialdehyds der Formel (III$_1$)

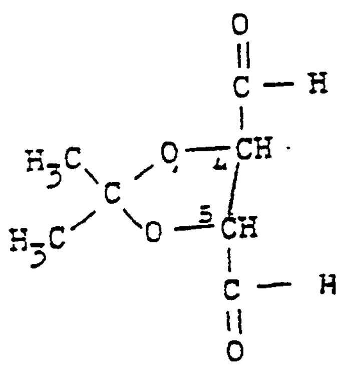

(III$_1$)

mit (4R, 5R)-Konfiguration zu gelangen, welchen man der Einwirkung eines Reagens der Formel

$$A-CH_2-\underset{\underset{O}{\|}}{C}-OR$$

unterzieht, worin A und R wie vorstehend definiert sind, um die Verbindung der Formel (IV″$_1$)

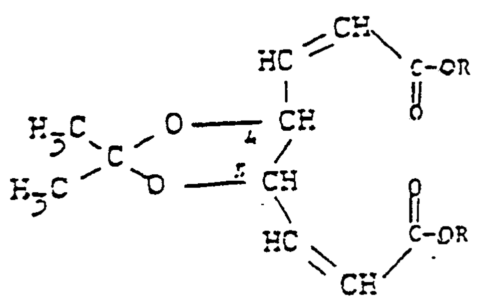

(IV″$_1$)

mit (4S, 5S)-Konfiguration zu erhalten, worin R wie vorstehend definiert ist und die Geometrie der Doppelbindungen die cis-Geometrie ist, welche man der Einwirkung eines Isopropylidendiphenylsulfurans unterzieht, um die Verbindung der Formel (V″$_1$)

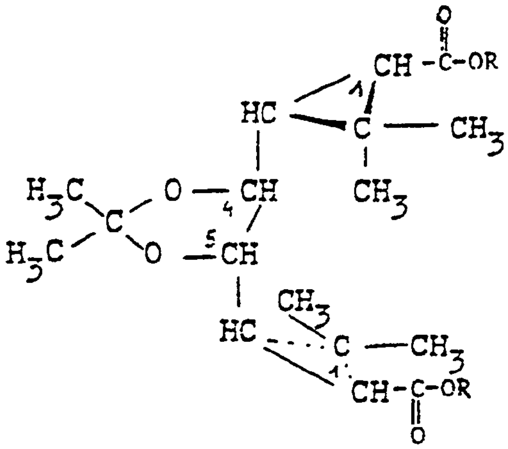

(V"₁)

mit (4S, 5S)- und (1R-cis)-Konfiguration im Hinblick auf die Cyclopropanringe zu erhalten, worin R wie vorstehend definiert ist, man entweder deren Dioxolanrest hydrolysiert, um die Verbindung der Formel (VI"$_1$)

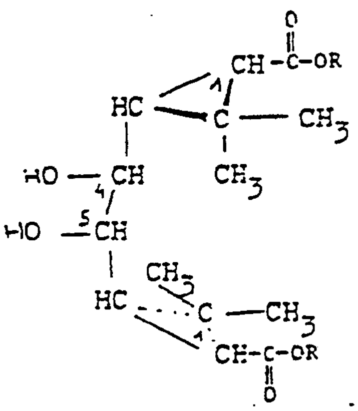

(VI"$_1$)

mit (4S, 5S)- und (1R-cis)-Konfiguration im Hinblick auf die Cyclopropanringe zu erhalten, worin R wie vorstehend definiert ist, und die 4,5-Bindung durch Einwirkung eines Oxidationsmittels spaltet, um die Verbindung der Formel ($I_B$) mit (1R -cis)-Konfiguration zu erhalten, oder den Dioxolanrest hydrolysiert und gleichzeitig die 4,5-Bindung spaltet, um die Verbindung der Formel ($I_B$) mit (1R-cis)-Konfiguration zu erhalten.

6. Die Verbindungen der Formel (IV), (V), (Va), (VI) und (VIa), wie in Anspruch 1 definiert.

7. Die durch Reduktion dem Diesters der Formel ($II_1$), wie in Anspruch 3 definiert, mit Diisobutylaluminiumhydrid erhaltene Verbindung.

**Patentansprüche für folgenden Vertragsstaat : ES**

1. Verfahren zur Herstellung von Verbindungen der Formel (I) :

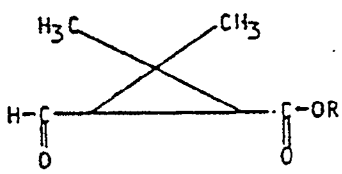

(I)

mit cis- oder trans-Struktur in racemischer oder optisch aktiver Form, worin R für ein Wasserstoffatom, einen Alkylrest mit 1 bis 4 Kohlenstoffatomen oder einen Arylrest mit bis zu 12 Kohlenstoffatomen steht, dadurch gekennzeichnet, daß man einen Diester der Formel (II) :

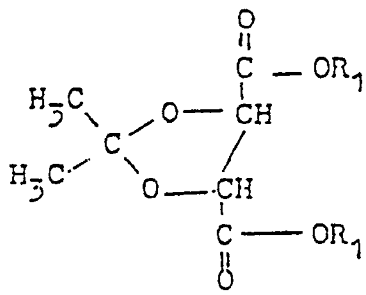

(II)

in irgendeiner seiner getrennten oder racemischen isomeren Formen, worin $R_1$ für einen Alkylrest mit 1 bis 4 Kohlenstoffatomen, einen Arylrest mit bis zu 12 Kohlenstoffatomen oder einen Aralkylrest mit bis zu 8 Kohlenstoffatomen steht, der Einwirkung eines Mittels für eine schonende Reduktion unterzieht, um zu einem Vorläufer eines Dialdehyds der Formel (III) :

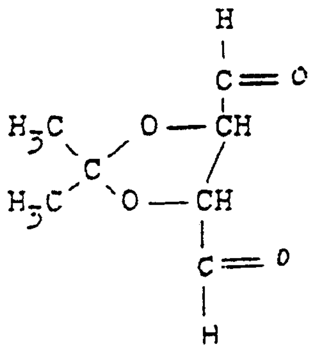

(III)

zu gelangen, welchen man der Einwirkung eines Wittig-Emmons-Horner-Reagens der Formel

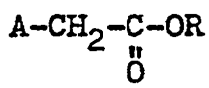

worin R wie vorstehend definiert ist und A den organischen Posphorrest dieses Reagens wiedergibt, unterzieht, um zu der Verbindung der Formel (IV)

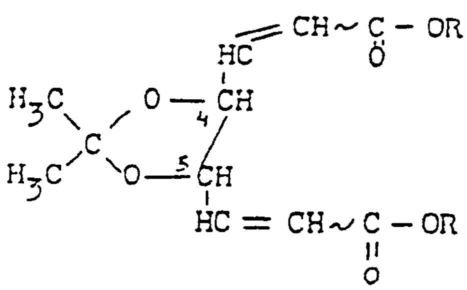

(IV)

zu gelangen, worin R wie vorstehend definiert ist und die gwellten Lienen eine cis- oder trans-Konfiguration schematisieren, welche man der Einwirkung eines Mittels für die gem-Dimethylcyclopropanierung unterzieht, um entweder zu der Verbindung der Formel (V)

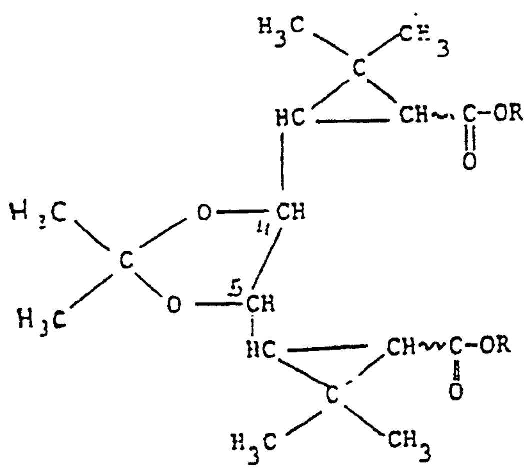

(V)

worin R und die gewellten Linien wie vorstehend definiert sind, oder zu der Verbindung der Formel (Va)

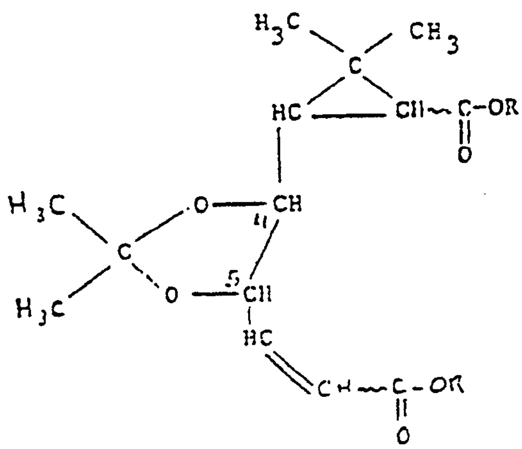

(Va)

zu gelangen, worin R und die gewellten Linien wie vorstehend definiert sind, welche Verbindung der Formel Va man gegebenenfalls der Einwirkung eines Mittels für die gem-Dimethylcyclopropanierung unterzieht, um zu der Verbindung der vorstehend definierten Formel (V) zu gelangen, man entweder den Dioxolanrest des Produkts der Formel (V) oder (Va) hydrolysiert, um entweder die Verbindung der Formel (VI)

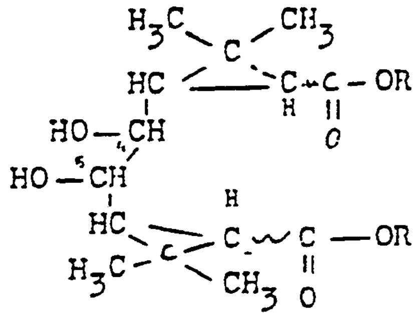

(VI)

worin R und die gewellten Linien wie vorstehend definiert sind, oder die Verbindung der Formel (VIa)

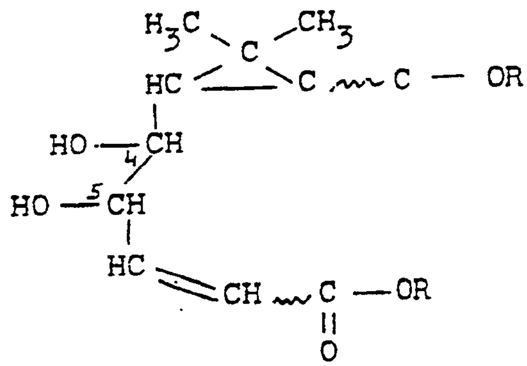

(VIa)

worin R und die gewellten Linien wie vorstehend definiert sind, zu erhalten, und die 4,5-Bindung der Verbindung der Formel (VI) oder (VIa) spaltet, um zu der erwarteten Verbindung der Formel (I) zu gelangen, oder den Dioxolanrest hydrolysiert und gleichzeitig die 4,5-Bindung spaltet, um die erwartete Verbindung der Formel (I) zu erhalten.

2. Verfharen gemäß Anspruch 1 zur Herstellung der Verbindungen der Formel ($I_A$)

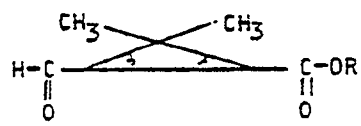

($I_A$)

mit racemischer oder optisch aktiver trans-Struktur, worin R wie vorstehend definiert ist, dadurch gekennzeichnet, daß man einen Diester der Formel (II), wie vorstehend definiert, in irgendeiner seiner getrennten oder racemischen isomeren Formen der Einwirkung eines Mittels zur schonenden Reduktion unterzieht, um zu einem Vorläufer für einen Dialdehyd der Formel (III), wie vorstehend definiert, mit der entsprechenden isomeren Struktur zu gelangen, welchen man der Einwirkung eines Reagens der Formel

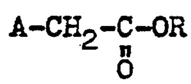

unterzieht, worin A und R wie vorstehend definiert sind, um zu der Verbindung der Formel (IV')

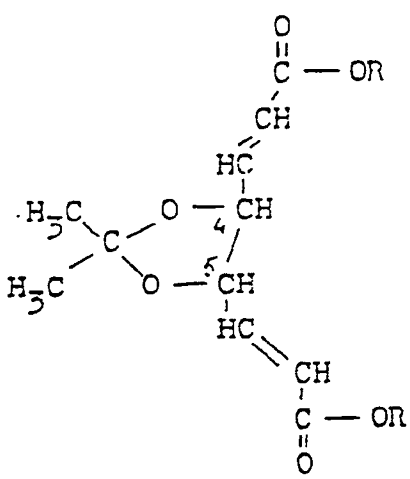

(IV')

zu gelangen, worin R wie vorstehend definiert ist sind die Geometrie der Doppelbindungen die trans-Geometrie ist, welche man der Einwirkung eines Mittels zur gem-Dimethylcyclopropanierung unterzieht, um zu der Verbindung der Formel (V')

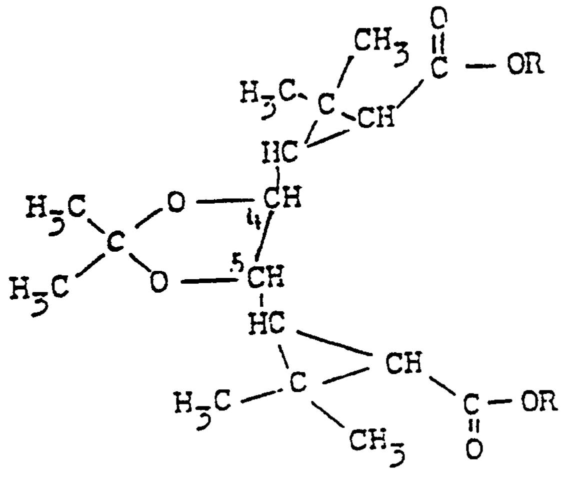

(V')

zu gelangen, worin R wie vorstehend definiert ist und die Konfiguration der Cyclopropanringe trans-ständig ist, man entweder deren Dioxolanrest hydrolysiert, um die Verbindung der Formel (VI')

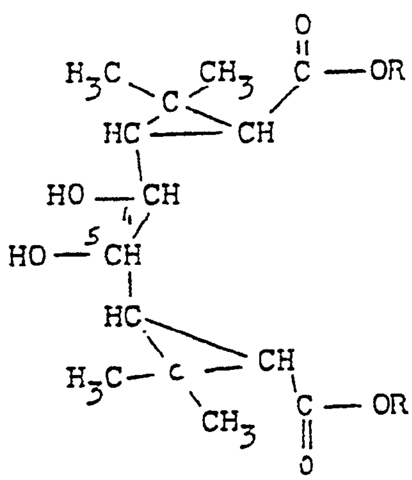

(VI')

zu erhalten, worin R und die Konfiguration der Cyclopropanringe wie vorstehend definiert sind, und die 4,5-Bindung spaltet, um die entsprechende Verbindung der Formel ($I_A$) zu erhalten, oder den Dioxolanrest hydrolysiert und gleichzeitig die 4,5-Bindung spaltet, um die erwartete Verbindung der Formel ($I_A$) zu erhalten.

3. Verfahren gemäß Anspruch 2 zur Herstellung der Verbindungen der Formel ($I_A$) mit 1R-trans-Konfiguration, dadurch gekennzeichnet, daß man einen Diester der Formel ($II_1$)

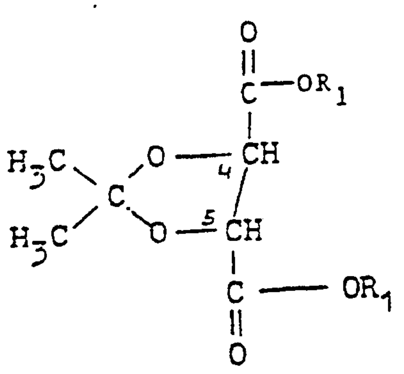

($II_1$)

mit (4R, 5R)-Konfiguration, worin $R_1$ wie vorstehend defiliert ist, der Einwirkung von Diisobutylaluminiumhydrid unterzieht, um zu dem entsprechenden Vorläufer des Dialdehyds der Formel ($III_1$)

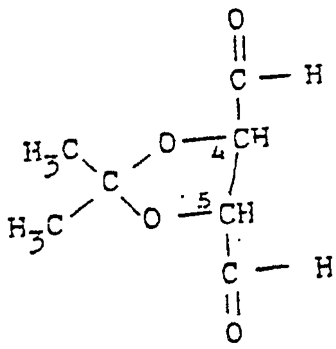

(III$_1$)

mit (4R, 5R)-Konfiguration zu gelangen, welchen man der Einwirkung eines Reagens der Formel

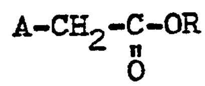

unterzieht, worin A und R wie vorstehend definiert sind, um zu der Verbindung der Formel (IV'$_1$)

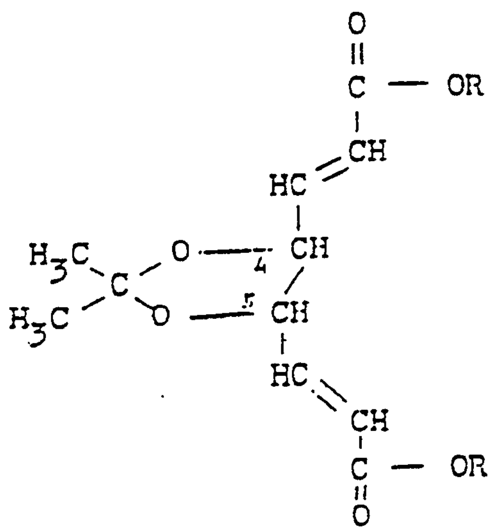

(IV'$_1$)

mit (4S, 5S)-Konfiguration zu gelagen, worin R wie vorstehend definiert ist und die Geometrie der Doppelbindungen die trans-Geometrie ist, welche man der Einwirkung eines Isopropylidentriphenylphosphorans unterzieht, um zu der Verbindung der Formel (V'$_1$)

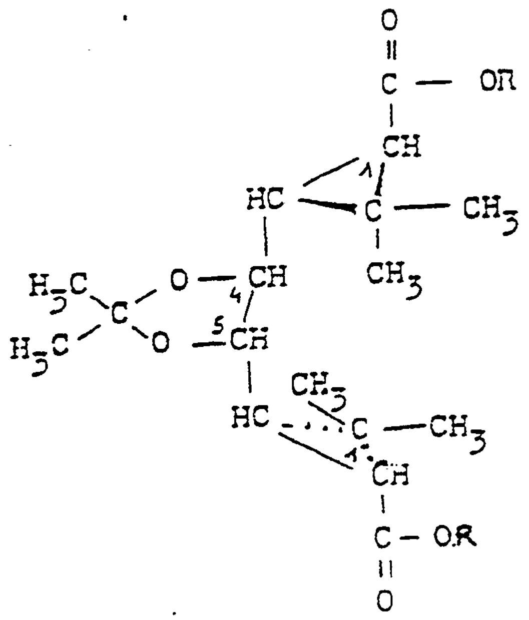

(V'$_1$)

mit (4S, 5S)- und (1R-trans)-Konfiguration hinsichtlich der Cyclopropanringe zu gelangen, worin R wie vorstehend definiert ist, man entweder deren Dioxolanrest hydrolysiert, um zu der Werbindung der Formel (VI'$_1$)

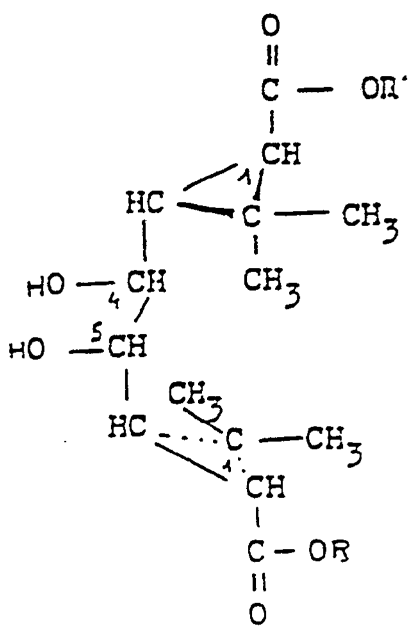

(VI'$_1$)

mit (4S, 5S)- und (1R-trans)-Konfiguration hinsichtlich der Cyclopropanringe zu gelangen, worin R wie vorstehend definiert ist, und die 4,5-Bindung durch ein Oxidationsmittel spaltet, um die Verbindung der Formel (I$_A$) mit (1R-trans)-Konfiguration zu erhalten, oder den Dioxolanrest hydrolysiert und gleichzeitig die 4,5-Bindung spaltet, um die Verbindung der Formel (I$_A$) mit (1R-trans)-Konfiguration zu erhalten.

4. Werfahren gemäß Anspruch 1 zur Herstellung von Verbindungen der Formel (I$_B$)

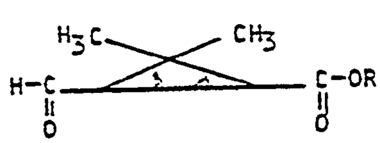

(I$_B$)

mit racemischer oder optisch aktiver cis-Struktur, worin R wie vorstehend definiert ist, dadurch gekennzeichnet, daß man einen Diester der Formel (II), wie vorstehend definiert, in irgendeiner seiner getrennten oder racemischen isomeren Formen der Einwirkung eines Mittels für die schonende Reduktion unterzieht, um zu einem Vorläufer eines Dialdehyds der Formel (III), wie vorstehend defiliert, mit entsprechender isomerer Struktur zu gelangen, welchen man der Einwirkung eines Reagens der Formel

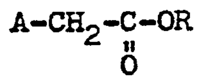

unterzieht, worin A und R wie vorstehend definiert sind, um zu der Verbindung der Formel (IV″)

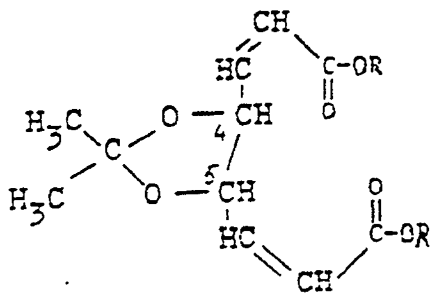

(IV″)

zu gelangen, worin R wie vorstehend definiert ist und die Geometrie der Doppelbindungen die cis-Geometrie ist, welche man der Einwirkung eines Isopropylidendiphenylsulfurans unterzieht, um die Verbindung der Formel (V″)

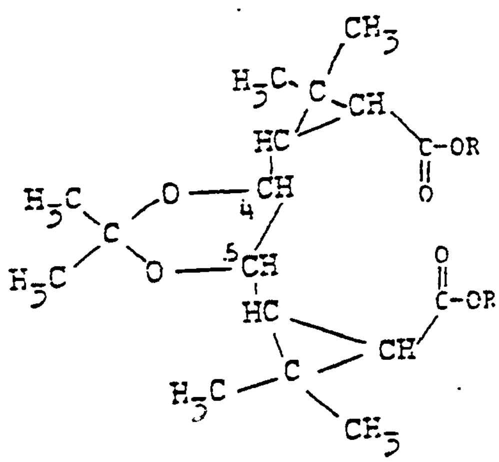

(V″)

zu erhalten, worin R wie vorstehend definiert ist und die Konfiguration der Cyclopropanringe die cis-Konfiguration ist, man entweder deren Dioxolanrest hydrolysiert,um die Verbindung der Formel (VI″)

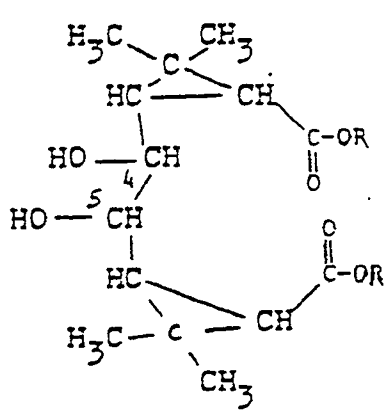

(VI″)

zu erhalten, worin R und die Konfiguration der Cyclopropanringe wie vorstehend definiert sind, und die 4,5-Bindung spaltet, um die entsprechende Verbindung der Formel ($I_B$) zu erhalten, oder den Dioxolanrest hydrolysiert und gleichzeitig die 4,5-Bindung spaltet, um die erwartete Verbindung der Formel ($I_B$) zu erhalten.

5. Verfahren gemäß Anspruch 4 zur Herstellung von Verbindungen der Formel ($I_B$) mit 1R-cis-Konfiguration, dadurch gekennzeichnet, daß man einen Diester der Formel ($II_1$)

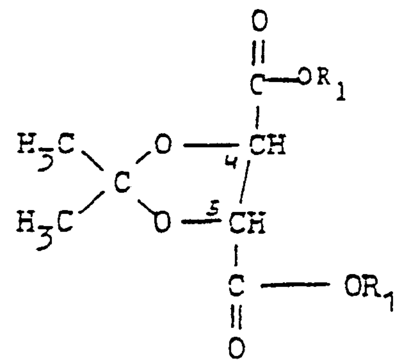

($II_1$)

mit (4R, 5R)-Konfiguration, worin $R_1$ wie vorstehend definiert ist, der Einwirkung von Diisobutylaluminiumhydrid unterzieht, um zu dem entsprechenden Vorlaüfer des Dialdehyds der Formel ($III_1$)

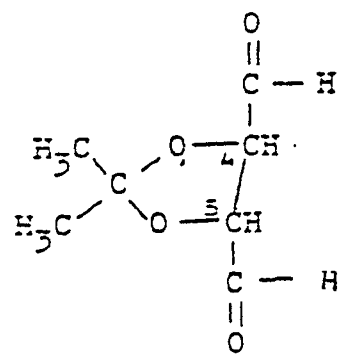

($III_1$)

mit (4R, 5R)-Konfiguration zu gelangen, welchen man der Einwirkung eines Reagens der Formel

$$A-CH_2-\underset{\underset{O}{\|}}{C}-OR$$

unterzieht, worin A und R wie vorstehend definiert sind, um die Verbindung der Formel ($IV''_1$)

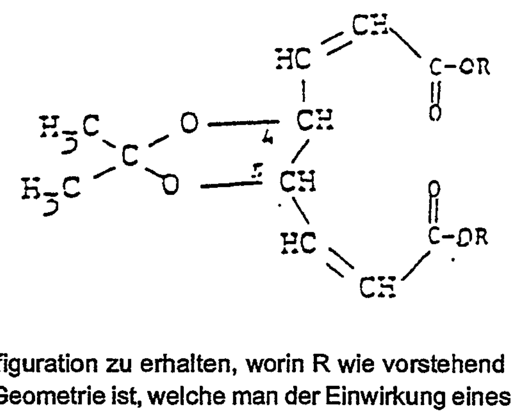

($IV''_1$)

mit (4S, 5S)-Kofiguration zu erhalten, worin R wie vorstehend definiert ist und die Geometrie der Doppelbindungen die cis-Geometrie ist, welche man der Einwirkung eines Isopropylidendiphenylsulfurans unterzieht, um die Verbindung der Formel ($V''_1$)

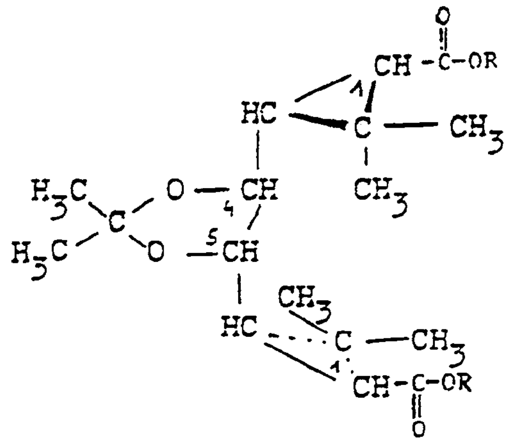

(V"$_1$)

mit (4S, 5S)- und (1R-cis)-Konfiguration im Hinblick auf die Cyclopropanringe zu erhalten, worin R wie vorstehend definiert ist, man entweder deren Dioxolanrest hydrolysiert, um die Verbindung der Formel (VI"$_1$)

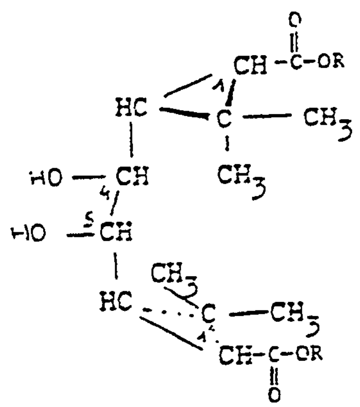

(VI"$_1$)

mit (4S, 5S)- und (1R-cis)-Konfiguration im Hinblick auf die Cyclopropanringe zu erhalten, worin R wie vorstehend definiert ist, und die 4,5-Bindung durch Einwirkung eines Oxidationsmittels spaltet, um die Verbindung der Formel ($I_B$) mit (1R -cis)-Konfiguration zu erhalten, oder den Dioxolanrest hydrolysiert und gleichzeitig die 4,5-Bindung spaltet, um die Verbindung der Formel ($I_B$) mit (1-cis)-Konfiguration zu erhalten.

## Claims

### Claims for the following Contracting states : AT, BE, CH, DE, FR, GB, IT, LI, LU, NL, SE

1. Preparation process for compounds of formula (I) :

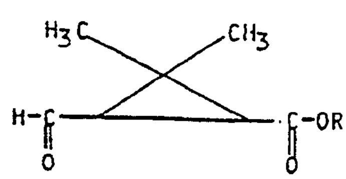

(I)

of cis or trans structure, racemic or optically active, in which R represents a hydrogen atom, an alkyl radical containing 1 to 4 carbon atoms or an aryl radical containing up to 12 carbon atoms, characterized in that a diester of formula (II) :

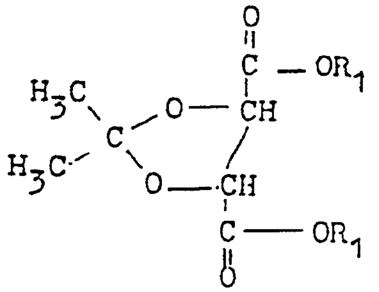

(II)

in any of its resolved or racemic isomer forms, in which $R_1$ represents an alkyl radical containing 1 to 4 carbon atoms, an aryl radical containing up to 12 carbon atoms or an aralkyl radical containing up to 8 carbon atoms, is subjected to the action of a reducing agent used sparingly in order to obtain a precursor of a dialdehyde of formula (III) :

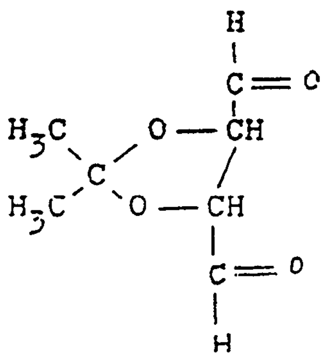

(III)

which is subjected to the action of a Wittig-Emmons-Horner reagent of formula :

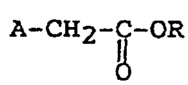

in which R is defined as previously and A represents the phosphorated organic remainder of the said reagent, in order to obtain the compound of formula (IV) :

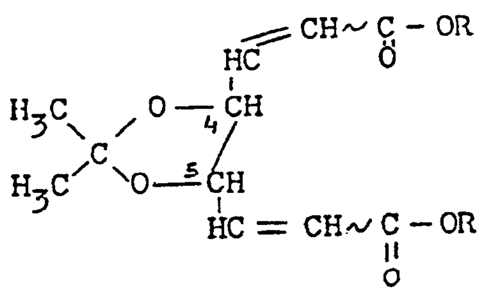

(IV)

in which R is defined as previously and the wavy lines outline a cis or trans configuration, which is subjected to the action of a gem-dimethyl cyclopropanation agent in order to obtain either the compound of formula (V):

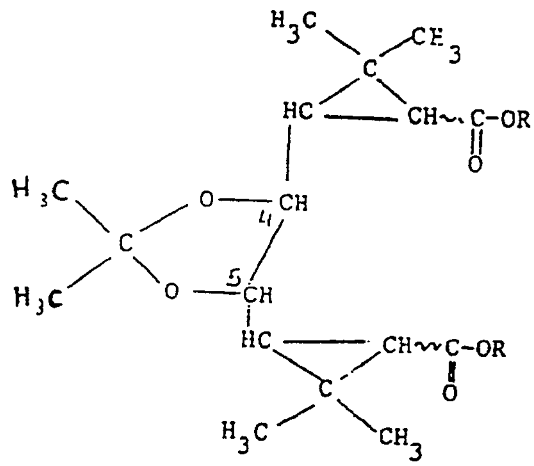

(V)

in which R and the wavy lines are defined as previously, or the compound of formula (Va) :

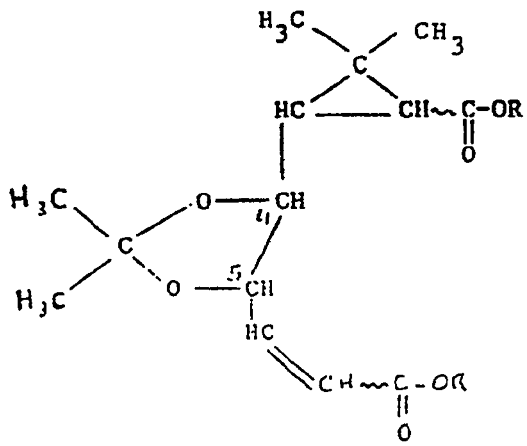

(Va)

in which R and the wavy lines are defined as previously, which compound of formula (Va), if appropriate, is subjected to a gem-dimethyl cyclopropanation agent, in order to obtain the compound of formula (V) defined previously, the dioxolane remainder of which product of formula (V) or (Va) either is hydrolyzed in order to obtain respectively either the compound of formula (VI) :

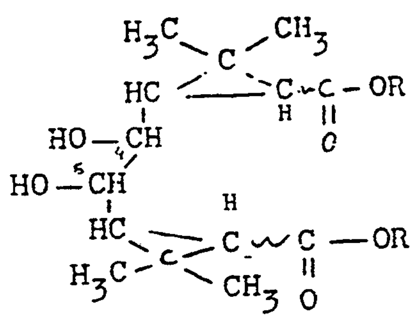

(VI)

in which R and the wavy lines are defined as previously, or the compound of formula (VIa) :

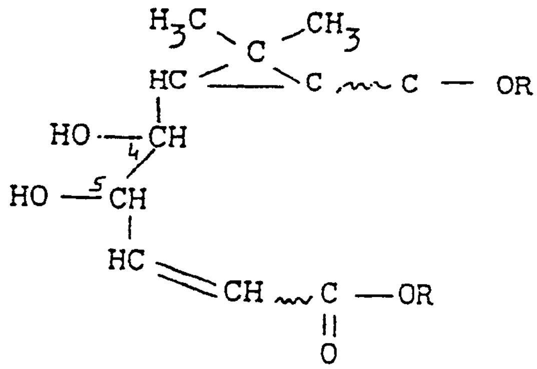

(VIa)

in which R and the wavy lines are defined as previously, the 4,5 bond of which compound of formula (VI) or (VIa) is cut in order to obtain the expected compound of formula (I), or the dioxolane remainder is hydrolyzed and the 4,5 bond is cut simultaneously, in order to obtain the expected compound of formula (I).

2. Process according to claim 1, for the preparation of compounds of formula ($I_A$) :

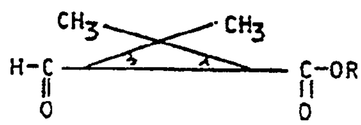

($I_A$)

of trans structure, racemic or optically active, in which R is defined as previously, characterized in that a diester of formula (II) as defined previously, in any of its resolved or racemic isomer forms, is subjected to the action of a reducing agent sparingly used, in order to obtain a precursor of a dialdehyde of formula (III) as defined previously, of corresponding isomer structure, which is subjected to the action of a reagent of formula :

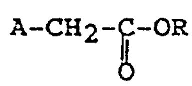

in which A and R are defined as previously, in order to obtain the compound of formula (IV') :

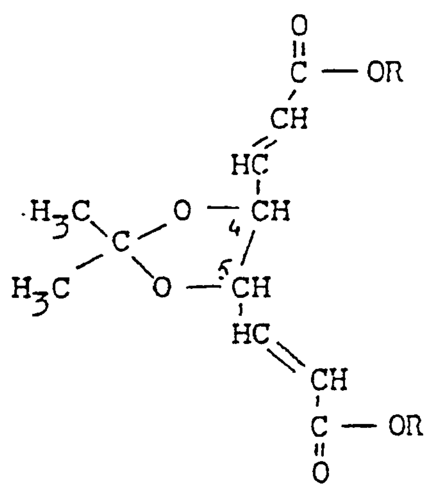

(IV')

in which R is defined as previously and the geometry of the double bonds is trans, which is subjected to the action of a gem-dimethyl cyclopropanation agent in order to obtain the compound of formula (V') :

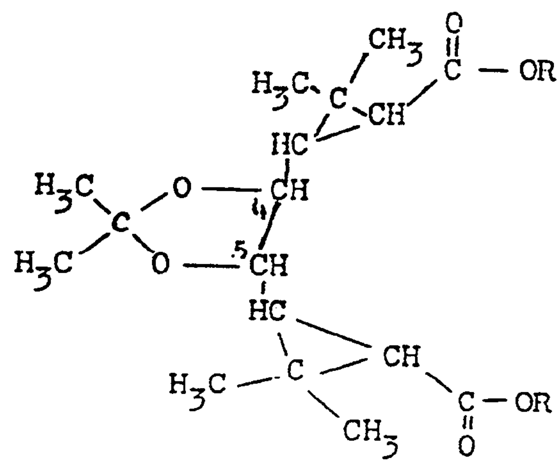

(V')

in which R is defined as previously and the configuration of the cyclopropane ring is trans, the dioxolane remainder of which either is hydrolyzed in order to obtain the compound of formula (VI') :

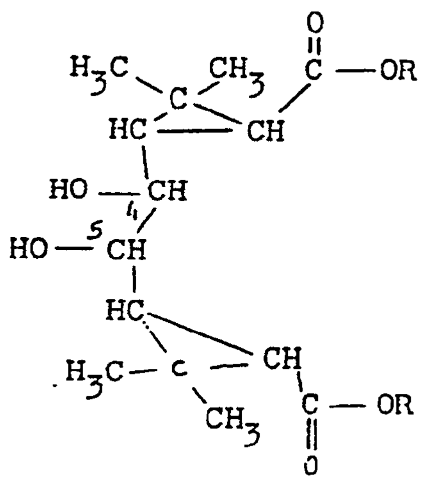

(VI')

in which R and the configurations of the cyclopropane rings are defined as previously, and the 4,5 bond is cut, in order to obtain the corresponding compound of formula ($I_A$), or the dioxolane remainder is hydrolyzed and the 4,5 bond is cut simultaneously in order to obtain the expected compound of formula ($I_A$).

3. Process according to claim 2, for the preparation of compounds of formula ($I_A$) of 1R trans configuration, characterized in that a diester of formula ($II_1$) :

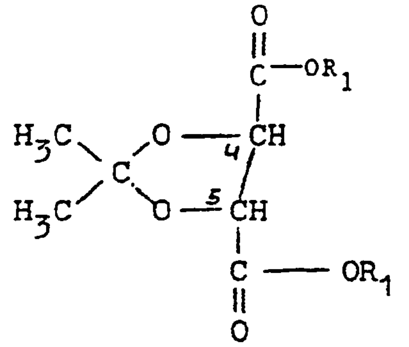

($II_1$)

of (4R, 5R) configuration, in which $R_1$ is defined as previously, is subjected to the action of a diisobutylaluminium hydride in order to obtain the corresponding precursor of the dialdehyde of formula ($III_1$) :

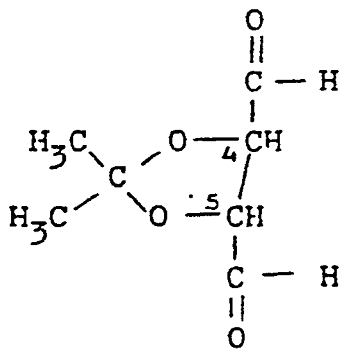

($III_1$)

of (4R, 5R) configuration, which is subjected to the action of a reagent of formula :

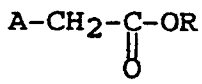

in which A and R are defined as previously, in order to obtain the compound of formula ($IV'_1$) :

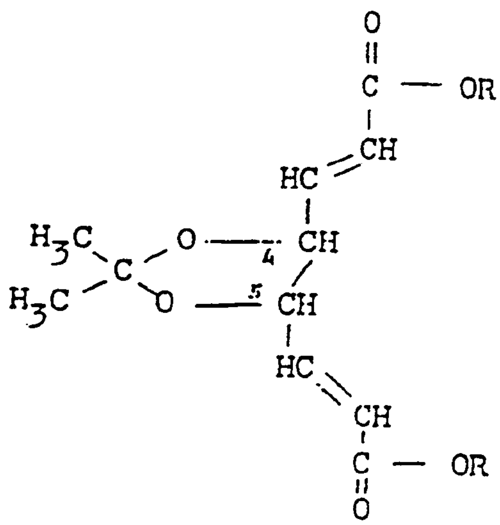

($IV'_1$)

of (4S, 5S) configuration, in which R is defined as previously and the geometry of the double bonds is <u>trans</u>, which is subjected to the action of an isopropylidene triphenylphosphorane in order to obtain the compound of formula ($V'_1$) :

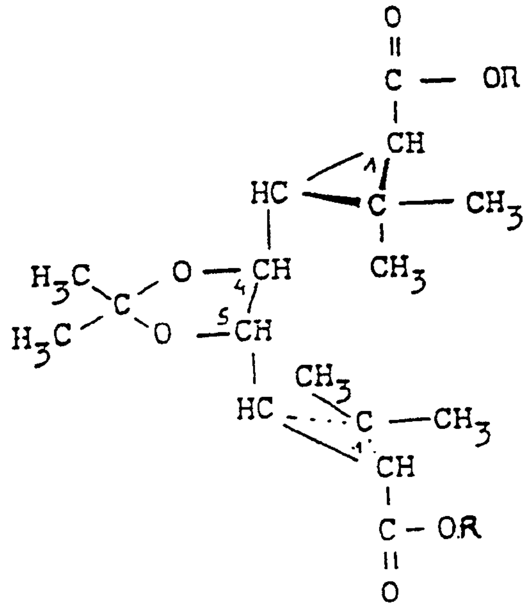

(V'$_1$)

of (4S, 5S) and (1R trans) configuration at the level of the cyclopropane rings, in which R is defined as previously, the dioxolane remainder of which either is hydrolyzed, in order to obtain the compound of formula (VI'$_1$):

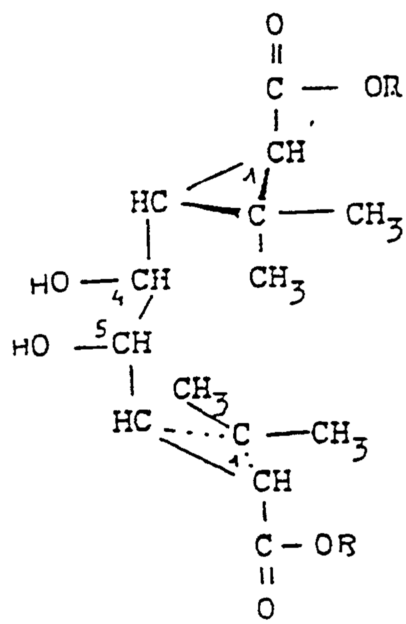

(VI'$_1$)

of (4S,5S and (1R trans) configuration at the level of the cyclopropane rings, in which R is defined as previously and the 4,5 bond is cut by the action of an oxidizing agent, in order to obtain the compound of formula ($I_A$) of (1R trans) configuration, or the dioxolane remainder is hydrolyzed and the 4,5 bond is cut simultaneously in order to obtain the compound of formula ($I_A$) of (1R trans) configuration.

4. Process according to claim 1, for the preparation of compounds of formula ($I_B$) :

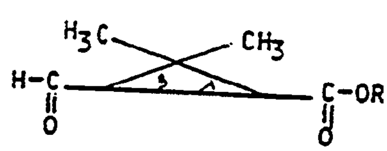

($I_B$)

of cis structure, racemic or optically active, in which R is defined as previously, characterized in that a diester

of formula (II) as defined previously, in any of its resolved or racemic forms, is subjected to the action of a reducing agent sparingly used, in order to obtain a precursor of a dialdehyde of formula (III) as defined previously, of corresponding isomer structure, which is subjected to the action of a reagent of formula :

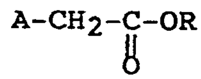

in which A and R are defined as previously, in order to obtain the compound of formula (IV") :

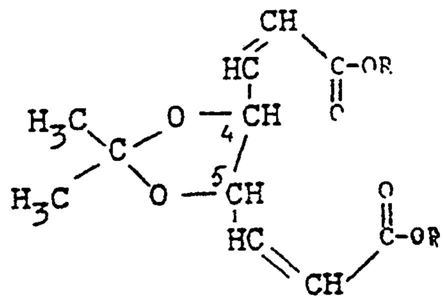

(IV")

in which R is defined as previously and the geometry of the double bonds is cis, which is subjected to the action of an isopropylidene diphenyl sulphurane in order to obtain the compound of formula (V") :

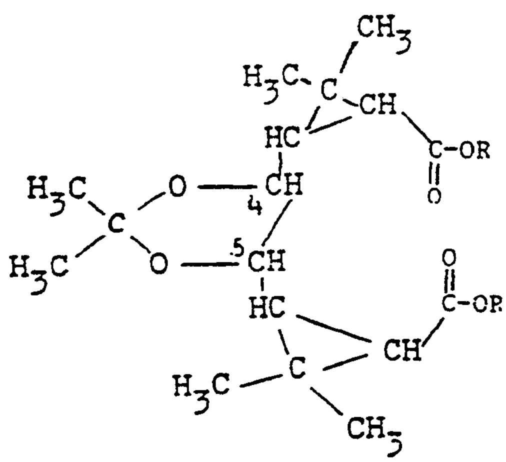

(V")

in which R is defined as previously and the configuration of the cyclopropane ring is cis, the dioxolane remainder of which either is hydrolyzed in order to obtain the compound of formula (VI") :

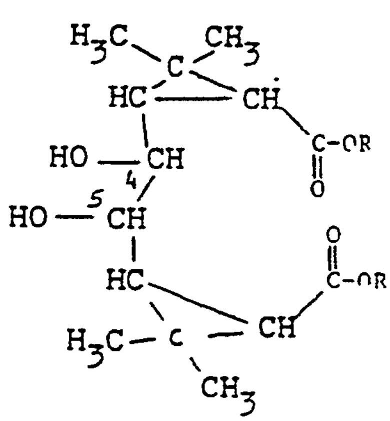

(VI")

in which R and the configuration of the cyclopropane rings are defined as previously, and the 4,5 bond is cut in order to obtain the corresponding compound of formula ($I_B$), or the dioxolane remainder is hydrolyzed and

the 4,5 bond is cut simultaneously, in order to obtain the expected compound of formula ($I_B$).

5. Process according to claim 4, for the preparation of compounds of formula ($I_B$) of 1R cis configuration, characterized in that a diester of formula ($II_1$) :

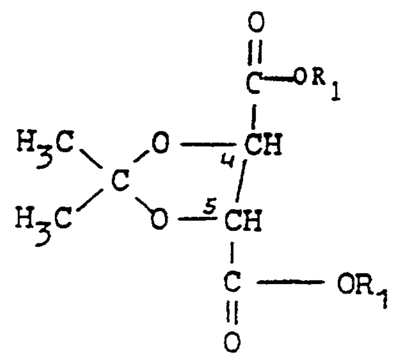

($II_1$)

of (4R, 5R) configuration, in which $R_1$ is defined as previously, is subjected to the action of diisobutylaluminium hydride in order to obtain the corresponding precursor of the dialdehyde of formula ($III_1$) :

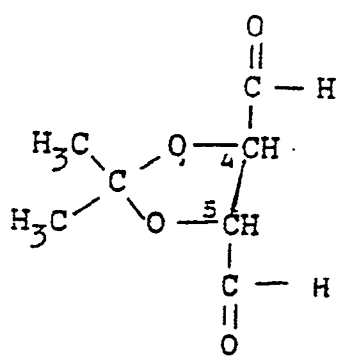

($III_1$)

of (4R, SR) configuration, which is subjected to the action of a reagent of formula :

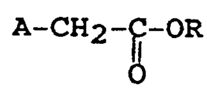

in which A and R are defined as previously, in order to obtain the compound of formula ($IV''_1$) :

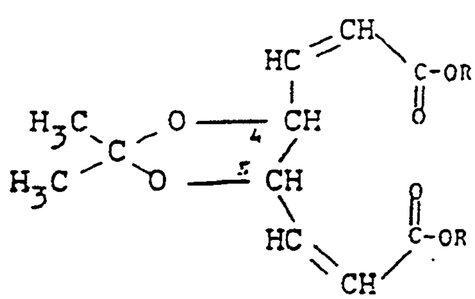

($IV''_1$)

of (4S, 5S) configuration, in which R is defined as previously and the geometry of the double bonds is cis, which is subjected to the action of an isopropylidene diphenylsulphurane in order to obtain the compound of formula ($V''_1$) :

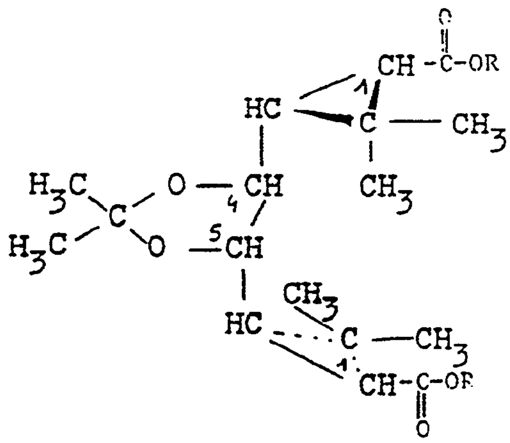

(V"1)

of (4S, 5S) and (1R cis) configuration at the cyclopropane rings, in which R is defined as previously, the dioxolane remainder of which either is hydrolyzed in order to obtain the compound of formula ($VI''_1$) :

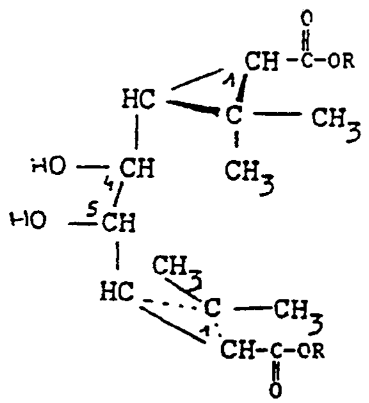

(VI"1)

of (4S, 5S) and (1R cis) configuration at the cyclopropane rings, in which R is defined as previously and the 4,5 bond is cut by the action of an oxidizing agent, in order to obtain the compound of formula ($I_B$) of (1R cis) configuration, or the dioxolane remainder is hydrolyzed and the 4,5 bond is simultaneously cut in order to obtain the compound of formula ($I_B$) of (1R cis) configuration.

6. The compounds of formulae (IV), (V), (Va), (VI) and (VIa) as defined in claim 1.

7. The compound as obtained by the reduction of the diester of formula ($II_1$) as defined in claim 3 by diisobutylaluminium hydride.

**Claims for the following Contracting state : ES**

1. Preparation process for compounds of formula (I) :

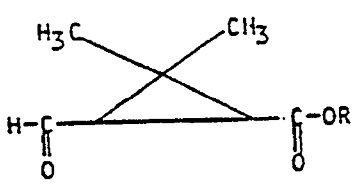

(I)

of cis or trans structure, racemic or optically active, in which R represents a hydrogen atom, an alkyl radical containing 1 to 4 carbon atoms or an aryl radical containing up to 12 carbon atoms, characterized in that a diester of formula (II) :

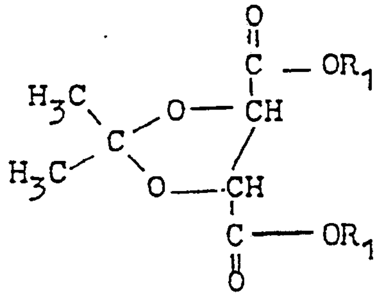

(II)

in any of its resolved or racemic isomer forms, in which $R_1$ represents an alkyl radical containing 1 to 4 carbon atoms, an aryl radical containing up to 12 carbon atoms or an aralkyl radical containing up to 8 carbon atoms, is subjected to the action of a reducing agent used sparingly in order to obtain a precursor of a dialdehyde of formula (III) :

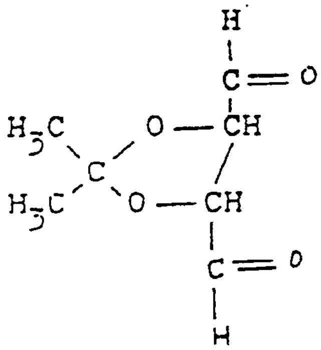

(III)

which is subjected to the action of a Wittig-Emmons-Horner reagent of formula :

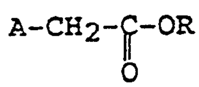

in which R is defined as previously and A represents the phosphorated organic remainder of the said reagent, in order to obtain the compound of formula (IV) :

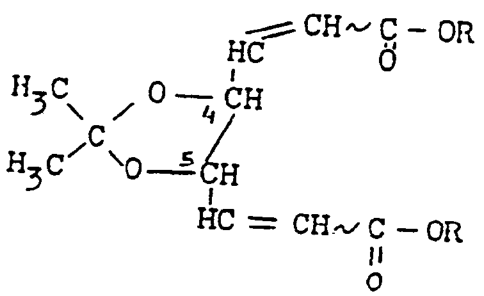

(IV)

in which R is defined as previously and the wavy lines outline a cis or trans configuration, which is subjected to the action of a gem-dimethyl cyclopropanation agent in order to obtain either the compound of formula (V):

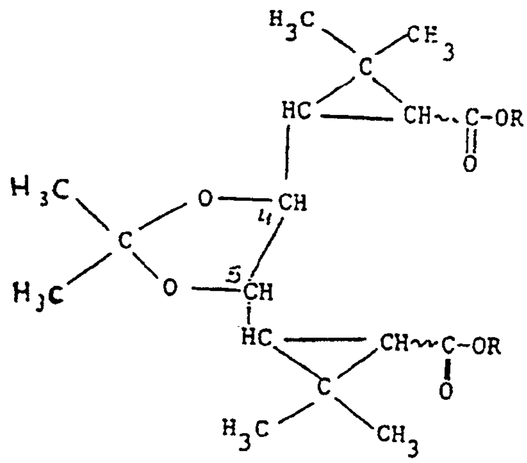

(V)

in which R and the wavy lines are defined as previously, or the compound of formula (Va) :

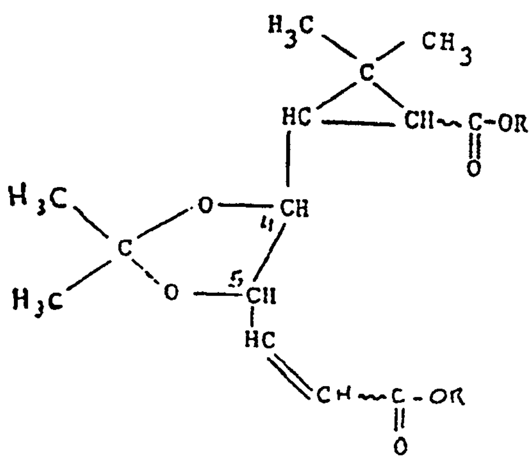

(Va)

in which R and the wavy lines are defined as previously, which compound of formula (Va), if appropriate, is subjected to a gem-dimethyl cyclopropanation agent, in order to obtain the compound of formula (V) defined previously, the dioxolane remainder of which product of formula (V) or (Va) either is hydrolyzed in order to obtain respectively either the compound of formula (VI) :

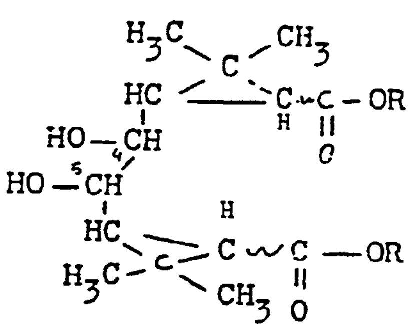

(VI)

in which R and the wavy lines are defined as previously, or the compound of formula (VIa) :

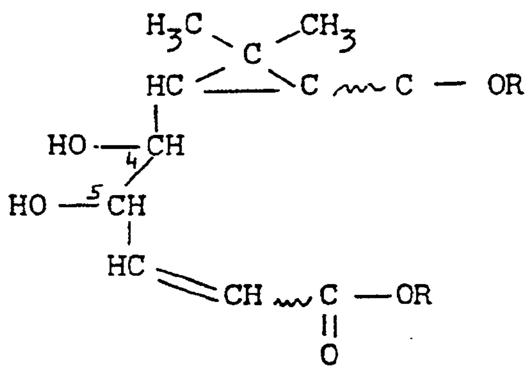

(VIa)

in which R and the wavy lines are defined as previously, the 4,5 bond of which compound of formula (VI) or (VIa) is cut in order to obtain the expected compound of formula (I), or the dioxolane remainder is hydrolyzed and the 4,5 bond is cut simultaneously, in order to obtain the expected compound of formula (I).

2. Process according to claim 1, for the preparation of compounds of formula ($I_A$) :

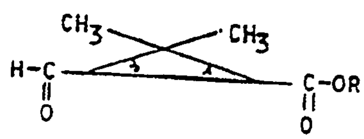

($I_A$)

of trans structure, racemic or optically active, in which R is defined as previously, characterized in that a diester of formula (II) as defined previously, in any of its resolved or racemic isomer forms, is subjected to the action of a reducing agent sparingly used, in order to obtain a precursor of a dialdehyde of formula (III) as defined previously, of corresponding isomer structure, which is subjected to the action of a reagent of formula :

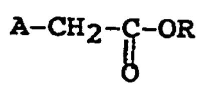

in which A and R are defined as previously, in order to obtain the compound of formula (IV') :

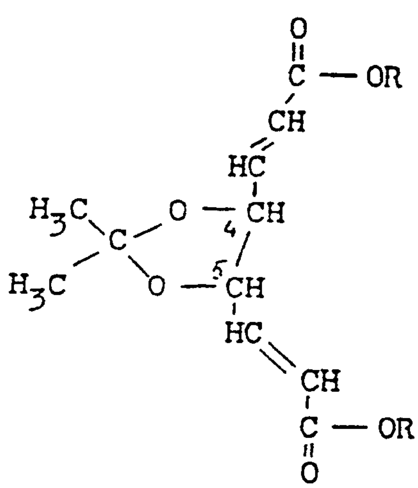

(IV')

in which R is defined as previously and the geometry of the double bonds is trans, which is subjected to the action of a gem-dimethyl cyclopropanation agent in order to obtain the compound of formula (V') :

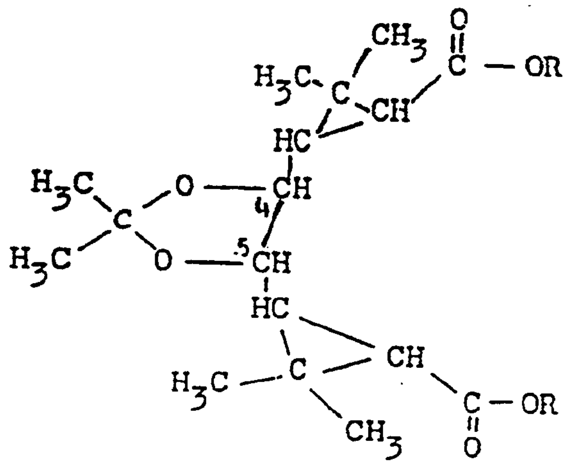

(V')

in which R is defined as previously and the configuration of the cyclopropane ring is trans, the dioxolane remainder of which either is hydrolyzed in order to obtain the compound of formula (VI') :

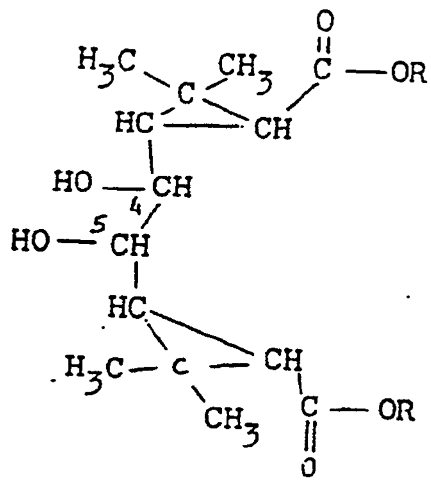

(VI')

in which R and the configurations of the cyclopropane rings are defined as previously, and the 4,5 bond is cut, in order to obtain the corresponding compound of formula ($I_A$),or the dioxolane remainder is hydrolyzed and the 4,5 bond is cut simultaneously in order to obtain the expected compound of formula ($I_A$).

3. Process according to claim 2, for the preparation of compounds of formula ($I_A$) of 1R trans configuration, characterized in that a diester of formula ($II_1$) :

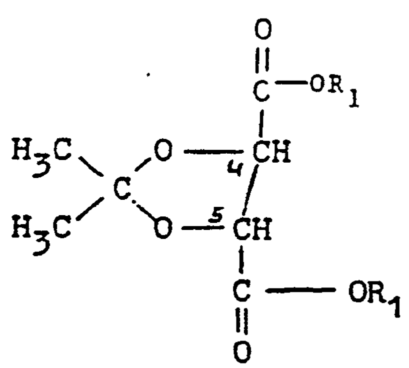

($II_1$)

of (4R, 5R) configuration, in which $R_1$ is defined as previously, is subjected to the action of a diisobutylaluminium hydride in order to obtain the corresponding precursor of the dialdehyde of formula ($III_1$) :

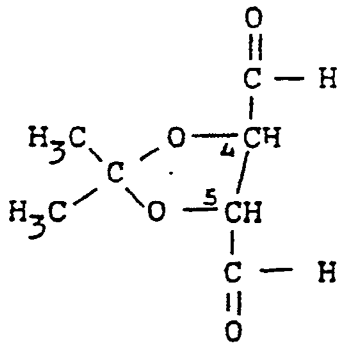

(III$_1$)

of (4R, 5R) configuration, which is subjected to the action of a reagent of formula :

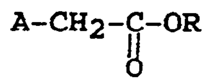

in which A and R are defined as previously, in order to obtain the compound of formula (IV'$_1$) :

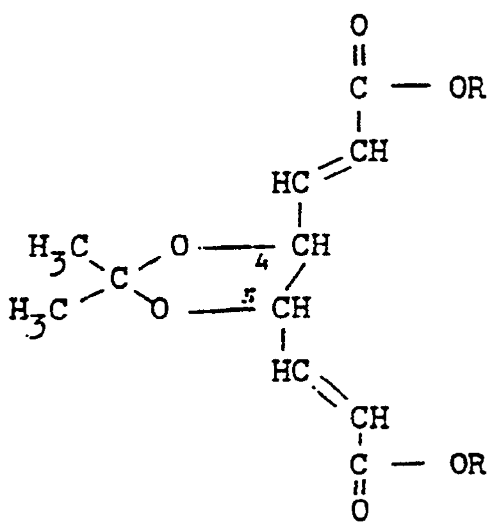

(IV'$_1$)

of (4S, 5S) configuration, in which R is defined as previously and the geometry of the double bonds is trans, which is subjected to the action of an isopropylidene triphenylphosphorane in order to obtain the compound of formula (V'$_1$) :

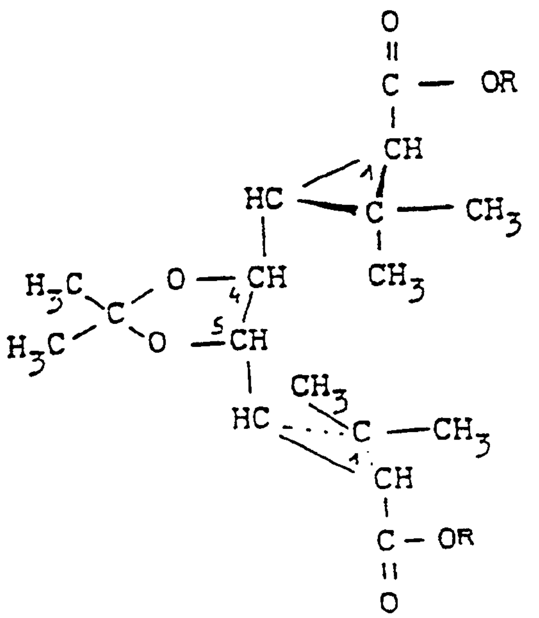

(V'1)

of (4S, 5S) and (1R trans) configuration at the level of the cyclopropane rings, in which R is defined as previously, the dioxolane remainder of which either is hydrolyzed, in order to obtain the compound of formula (VI'$_1$):

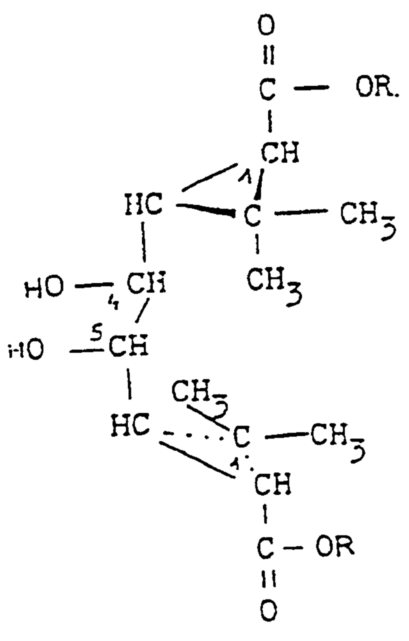

(VI'1)

of (4S, 5S and (1R trans) configuration at the level of the cyclopropane rings, in which R is defined as previously and the 4,5 bond is cut by the action of an oxidizing agent, in order to obtain the compound of formula ($I_A$) of (1R trans) configuration, or the dioxolane remainder is hydrolyzed and the 4,5 bond is cut simultaneously in order to obtain the compound of formula ($I_A$) of (1R trans) configuration.

4. Process according to claim 1, for the preparation of compounds of formula ($I_B$) :

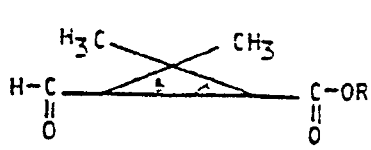

($I_B$)

of cis structure, racemic or optically active, in which R is defined as previously, characterized in that a diester of formula (II) as defined previously, in any of its resolved or racemic forms, is subjected to the action of a reducing agent sparingly used, in order to obtain a precursor of a dialdehyde of formula (III) as defined previously, of corresponding isomer structure, which is subjected to the action of a reagent of formula :

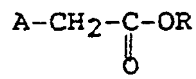

in which A and R are defined as previously, in order to obtain the compound of formula (IV") :

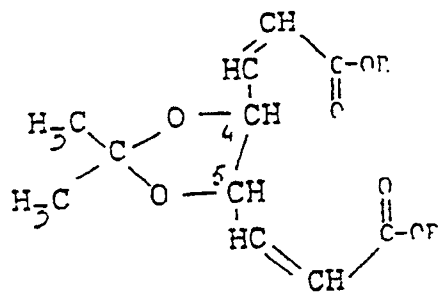

(IV")

in which R is defined as previously and the geometry of the double bonds is cis, which is subjected to the action of an isopropylidene diphenyl sulphurane in order to obtain the compound of formula (V") :

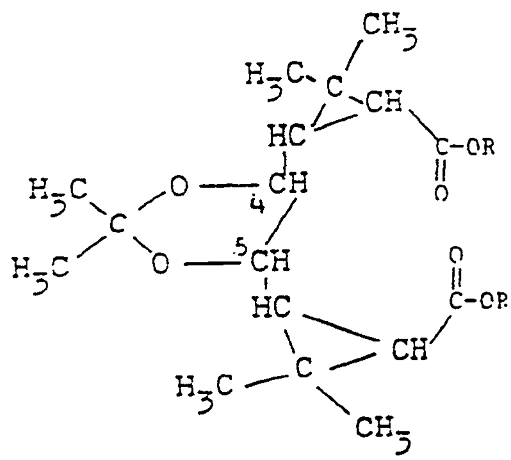

(V")

in which R is defined as previously and the configuration of the cyclopropane ring is cis, the dioxolane remainder of which either is hydrolyzed in order to obtain the compound of formula (VI") :

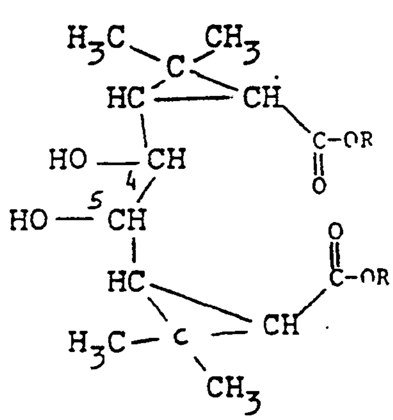

(VI")

in which R and the configuration of the cyclopropane rings are defined as previously, and the 4,5 bond is cut in order to obtain the corresponding compound of formula ($I_B$), or the dioxolane remainder is hydrolyzed and the 4,5 bond is cut simultaneously, in order to obtain the expected compound of formula ($I_B$).

5. Process according to claim 4, for the preparation of compounds of formula ($I_B$) of 1R cis configuration, characterized in that a diester of formula ($II_1$) :

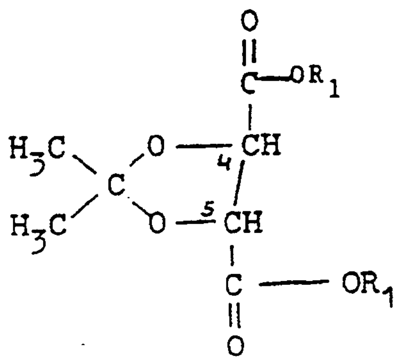

($II_1$)

of (4R, 5R) configuration, in which $R_1$ is defined as previously, is subjected to the action of diisobutylaluminium hydride in order to obtain the corresponding precursor of the dialdehyde of formula ($III_1$) :

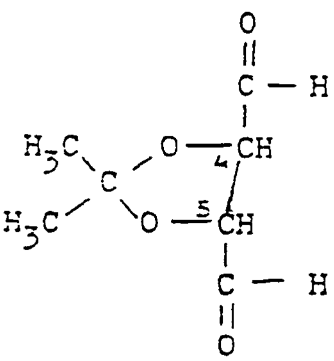

($III_1$)

of (4R, 5R) configuration, which is subjected to the action of a reagent of formula :

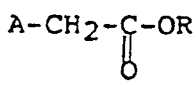

in which A and R are defined as previously, in order to obtain the compound of formula ($IV''_1$) :

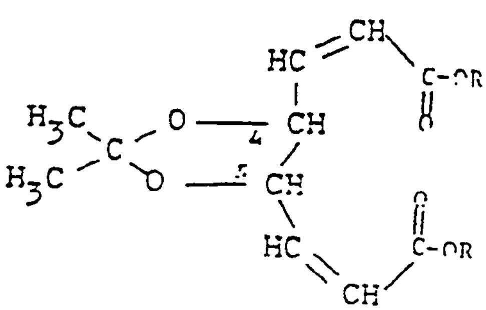

($IV''_1$)

of (4S, 5S) configuration, in which R is defined as previously and the geometry of the double bonds is cis, which is subjected to the action of an isopropylidene diphenylsulphurane in order to obtain the compound of formula ($V''_1$) :

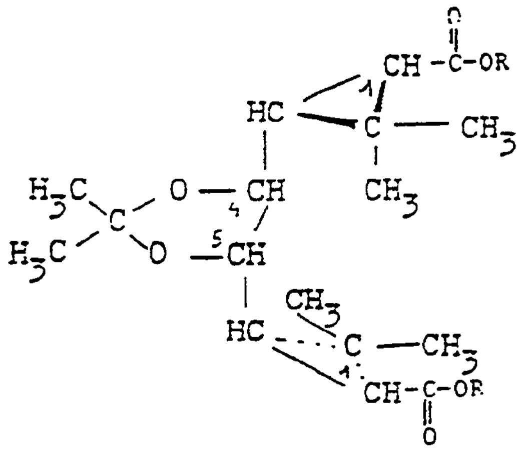

(V"$_1$)

of (4S, 5S) and (1R cis) configuration at the cyclopropane rings, in which R is defined as previously, the dioxolane remainder of which either is hydrolyzed in order to obtain the compound of formula (VI"$_1$) :

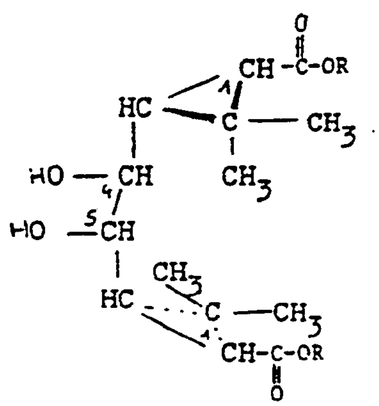

(VI"$_1$)

of (4S, 5S) and (1R cis) configuration at the cyclopropane rings, in which R is defined as previously and the 4,5 bond is cut by the action of an oxidizing agent, in order to obtain the compound of formula ($I_B$) of (1R cis) configuration, or the dioxolane remainder is hydrolyzed and the 4,5 bond is simultaneously cut in order to obtain the compound of formula ($I_B$) of (1R cis) configuration.